(19) Europäisches Patentamt / European Patent Office / Office européen des brevets

(11) **EP 3 829 756 B1**

(12) **EUROPEAN PATENT SPECIFICATION**

(45) Date of publication and mention
of the grant of the patent:
**03.09.2025 Bulletin 2025/36**

(51) International Patent Classification (IPC):
**A61Q 17/04** $^{(2006.01)}$    **B01J 13/02** $^{(2006.01)}$
**B01J 13/20** $^{(2006.01)}$

(21) Application number: **19845555.2**

(22) Date of filing: **22.07.2019**

(52) Cooperative Patent Classification (CPC):
**B01J 13/02; A61K 8/0241; A61K 8/29;**
**A61Q 17/04; B01J 13/20;** A61K 2800/412;
A61K 2800/413; A61K 2800/622; A61K 2800/651;
A61K 2800/652

(86) International application number:
**PCT/SG2019/050352**

(87) International publication number:
**WO 2020/027722 (06.02.2020 Gazette 2020/06)**

(54) **JANUS PARTICLE PREPARATION THROUGH TWO-PHASE INTERFACE ASSEMBLY**

JANUS-PARTIKELPRÄPARAT DURCH ZWEIPHASENGRENZFLÄCHENANORDNUNG

PRÉPARATION DE PARTICULES JANUS AU MOYEN D'UN ENSEMBLE INTERFACE À DEUX
PHASES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **02.08.2018 SG 10201806616T**
**25.03.2019 SG 10201902629Y**

(43) Date of publication of application:
**09.06.2021 Bulletin 2021/23**

(73) Proprietors:
• **Nanyang Technological University**
**Singapore 639798 (SG)**
• **Kenvue Brands LLC**
**Summit, NJ 07901 (US)**

(72) Inventors:
• **CHEN, Zhong**
**Singapore 639798 (SG)**
• **TAN, Si Jia Jasmine**
**Singapore 639798 (SG)**

(74) Representative: **Marks & Clerk LLP**
**15 Fetter Lane**
**London EC4A 1BW (GB)**

(56) References cited:
**US-A1- 2008 234 394**

• XIAONING FU ET AL: "Arrays of AuTiOJanus-like
nanoparticles fabricated by block copolymer
templates and their photocatalytic activity in the
degradation of methylene blue", MATERIALS
CHEMISTRY AND PHYSICS, ELSEVIER SA,
SWITZERLAND, TAIWAN, REPUBLIC OF CHINA,
vol. 130, no. 1, 25 June 2011 (2011-06-25), pages
334 - 339, XP028389140, ISSN: 0254-0584,
[retrieved on 20110701], DOI: 10.1016/
J.MATCHEMPHYS.2011.06.054
• LIU LIANYING ET AL: "Preparation of Polymeric
Janus Particles by Directional UV-Induced
Reactions", LANGMUIR, vol. 25, no. 18, 15
September 2009 (2009-09-15), US, pages 11048 -
11053, XP055903151, ISSN: 0743-7463, DOI:
10.1021/la901364a
• ZENERINO, A. ET AL.: "Synthesis of fluorinated
ceramic Janus particles via a Pickering emulsion
method", JOURNAL OF COLLOID AND
INTERFACE SCIENCE, vol. 450, 16 March 2015
(2015-03-16), pages 174 - 181, XP055454126,
[retrieved on 20190917], DOI: 10.1016/
j.jcis.2015.03.011

- TAN, J. S. J. ET AL.: "Mask-less preparation of Janus particles through ultraviolet irradiation on hydrophobic particles assembled at the air-water interface", JOURNAL OF COLLOID AND INTERFACE SCIENCE, vol. 546, 25 March 2019 (2019-03-25), pages 285 - 292, XP085659067, [retrieved on 20190917], DOI: 10.1016/ j.jcis.2019.03.081

**Description**

**Field of Invention**

The invention relates to a Janus particle comprising a core inorganic photocatalytic particle, a method of making said particle, and the use of said particle in sunscreen formulations.

**Background**

The listing or discussion of a prior-published document in this specification should not necessarily be taken as an acknowledgement that the document is part of the state of the art or is common general knowledge.

The creation of asymmetric, or Janus particles has been a field of interest since its conceptualisation by De Gennes in 1991. Such particles contain two (or more) distinct chemical or physical properties on their surface, or throughout their core. The asymmetry of such particles conveys directionality which facilitates its self-assembly depending on the environment it is placed in. In contrast, such directionality is typically absent in homogeneous particles.

Janus particles may be synthesised bottom-up, or existing particles may be modified to become Janus-like, i.e. *via* top-down method. Many methods to attain Janus particles have been conceived, and these particles may be comprised of polymeric or inorganic components, or sometimes both (Hu, J. et al., Chem. Soc. Rev. 2012, 41, 4356-4378). An intuitive modification method may be based on toposelective surface modification, and this is a process to make Janus particles amongst many others (Perro, A. et al., J. Mater. Chem. 2005, 15, 3745-3760). Various strategies have also been conceived, such as temporary masking, microcontact printing, partial contact with a reactive medium along an interface, and using reactive directional fluxes or fields.

Present technologies to make Janus particles largely involve masking, which protects one area of the particle while subjecting the other area to modification. A well-known technique involves paraffin wax masking, which is capable of producing gram quantities of Janus particles in a single batch (US 20080234394 A1). In this method, Pickering emulsions were made out of silica particles and paraffin wax, which functioned as a masking surface and enabled only the exposed areas of the particles to be modified by aminopropylsilane (APS). It was demonstrated that the incorporation and control of the concentration of the surfactants has to be precise as it could affect the amount of particle area subjected to modification (Jiang, S.; Granick, S., Langmuir 2008, 24, 2438-2445). However, such techniques necessitate the use of a mask, which is an additional component that has to be removed in the work-up.

A method that does not require an additional masking surface was conceived by Takahara *et* al., which involves particle-particle masking (Takahara, Y. K. et al., J. Am. Chem. Soc. 2005, 127, 6271-6275). This method relies on the agglomeration of silica particles in an immiscible water-toluene system to partially modify the exposed surface of the particles with *n*-octadecyltrimethoxysilane, while keeping the agglomerated surfaces within untouched. However, the addition of a defined, small amount of water to facilitate a tight aggregation of the silica particles requires precise control of the reaction conditions and is not feasible for large-scale production. Therefore, a main difficulty in the synthesis of Janus particles is to achieve large-scale production, with high yield and particle uniformity.

Given the above, there remains a need to develop new synthesis methods that produce Janus particles more efficiently with high yields, and at the same time achieve high particle uniformity and monodispersibility. More importantly, these methods have to be robust, cost-effective and versatile, such that Janus particles with various morphologies can be achieved easily.

**Summary of Invention**

As noted above, there remains a need for improved Janus particles, as well as methods of manufacture thereof.

Thus, in a first aspect of the invention, there is disclosed a Janus particle comprising:

a core inorganic photocatalytic particle having a surface with a first region and a second region and an average diameter of from 50 nm to 10 $\mu$m; and

a $C_8$ to $C_{26}$ alkylsilane or a fluoroalkylsilane coating that is susceptible to photo-degradation, which coating is covalently bound to the surface of the core inorganic photocatalytic particle where present, wherein:

the coating fully coats the entire surface of the first region and the second region is coated by a residue formed by photodegradation of the coating, such that the first region displays hydrophobic or hydrophobic and oleophobic properties and the second region displays amphiphilic properties.

In embodiments of the first aspect of the invention:

(i) the core inorganic photocatalytic particle may be selected from one or more of $CeO_2$, $SnO_2$, $Nb_2O_5$, $WO_3$, $Fe_2O_3$,

$Ta_2O_3$, CuO, NiO, $Cr_2O_3$, $RuO_2$, $TiO_2$, ZnO, and $Cu_2O$ (optionally wherein the core inorganic photocatalytic particle may be selected from one or more of $TiO_2$, ZnO, and $Cu_2O$ (e.g. the core inorganic photocatalytic particle may be $TiO_2$)) and/or the core inorganic photocatalytic particle may itself be a hybrid core-shell particle having a core selected from one or more of silica, a polymer, a ceramic, a metal and an alloy and a shell comprising one or more of $CeO_2$, $SnO_2$, $Nb_2O_5$, $WO_3$, $Fe_2O_3$, $Ta_2O_3$, CuO, NiO, $Cr_2O_3$, $RuO_2$, $TiO_2$, ZnO, and $Cu_2O$;

(ii) the coating is a low surface energy organic coating selected from octylsilane, hexadecylsilane, 1H,1H,2H,2H-perfluorodecylsilane covalently bound to the surface of the core inorganic photocatalytic particle, or it may be a $C_6$ to $C_{26}$ fluoroalkylsilane, such as 1H,1H,2H,2H-perfluorodecylsilane);

(iii) the first region may form from 30 to 70% of the surface area of the Janus particle and the second region forms from 70 to 30% of the surface area of the Janus particle, optionally wherein the first region may form from 40 to 60% of the surface area of the Janus particle and the second region forms from 60 to 40% of the surface area of the Janus particle, optionally wherein the first and second region form about 50% of the surface area of the Janus particle;

(iv) the Janus particles, when in the form of a compact film, have a water contact angle of from 30 to 140°.

In a second aspect of the invention, there is provided a method of manufacturing a Janus particle, the method comprising the steps of:

(a) providing an aqueous dispersion of precursor Janus particles comprising a core inorganic photocatalytic particle, the core inorganic photocatalytic particle having an average diameter of from 50 nm to 10 μm, wherein the core inorganic photocatalytic particle has a surface that is fully coated by a $C_8$ to $C_{26}$ alkylsilane or a fluoroalkylsilane coating that is susceptible to photo-degradation, which coating is covalently bound to the surface of the core inorganic photocatalytic particle; and

(b) subjecting the aqueous dispersion to irradiation by ultra-violet light for a period of time to provide Janus particles comprising:

the core inorganic photocatalytic particle having a surface with a first region and a second region; and a $C_8$ to $C_{26}$ alkylsilane or a fluoroalkylsilane coating that is susceptible to photo-degradation, which coating is covalently bound to the surface of the core inorganic photocatalytic particle where present, wherein:

the coating fully coats the entire surface of the first region and the second region is coated by a residue formed by photo-degradation of the coating, such that; the first region displays hydrophobic or hydrophobic and oleophobic properties; and the second region displays amphiphilic properties, wherein the period of time is from 30 minutes to 2 hours; the ultra-violet light is provided at a wavelength of from 100 to 400 nm; and the ultra-violet light is provided at an irradiance value of from 50 to 150 mW/cm$^2$.

In embodiments of the second aspect of the invention:

(ai) the period of time may be 1 hour;

(aii) the ultra-violet light may be provided at a wavelength of 365 nm;

(aiii) the ultra-violet light may be provided at an irradiance value of from 70 to 140 mW/cm$^2$ (e.g. 72 mW/cm$^2$ or 136 mW/cm$^2$);

(aiv) the precursor Janus particles may be prepared by reacting core inorganic photocatalytic particles with one or more of a $C_8$ to $C_{26}$ alkyltrialkoxysilane and a fluoroalkyltrialkoxysilane to provide the precursor Janus particles, optionally wherein the core inorganic photocatalytic particles are reacted with one or more of octyltriethoxysilane, hexadecyltrimethoxysilane, and 1H,1H,2H,2H-perfluoro-decyltriethoxysilane;

(av) the precursor Janus particles may be partially coated in a wax that covers from 30 to 70%, such as from 60 to 40% (e.g. 50%) of the surface area of the precursor Janus particle, optionally wherein after step (b) the wax is removed from the particles using a suitable organic solvent;

(avi) the precursor Janus particles are provided in the form of water-in-oil Pickering emulsion droplets that are dispersed into water to form the aqueous dispersion of precursor Janus particles, optionally wherein the low surface energy organic coating is a fluoroalkylsilane (e.g. 1H,1H,2H,2H-perfluorodecylsilane) covalently bound to the surface of the core inorganic photocatalytic particle;

(avii) the core inorganic photocatalytic particle may be selected from one or more of $CeO_2$, $SnO_2$, $Nb_2O_5$, $WO_3$, $Fe_2O_3$, $Ta_2O_3$, CuO, NiO, $Cr_2O_3$, $RuO_2$, $TiO_2$, ZnO, and $Cu_2O$ optionally wherein the core inorganic photocatalytic particle may be selected from one or more of $TiO_2$, ZnO, and $Cu_2O$ (e.g. the core inorganic photocatalytic particle is $TiO_2$) and/or the core inorganic photocatalytic particle may itself be a hybrid core-shell particle having a silica core and a shell comprising one or more of $CeO_2$, $SnO_2$, $Nb_2O_5$, $WO_3$, $Fe_2O_3$, $Ta_2O_3$, CuO, NiO, $Cr_2O_3$, $RuO_2$, $TiO_2$, ZnO, and $Cu_2O$; (aviii) the coating is a low surface energy organic coating is selected from octylsilane, hexadecylsilane, 1H,1H,2H,2H-perfluorodecylsilane covalently bound to the surface of the core inorganic photocatalytic particle (e.g. the low surface energy organic coating may be a $C_6$ to $C_{26}$ fluoroalkylsilane, such as 1H,1H,2H,2H-perfluorodecylsilane, covalently bound to the surface of the core inorganic photocatalytic particle).

In a third aspect of the invention, there is provided a sunscreen formulation comprising Janus particles as described in the first aspect of the invention and a diluent, carrier or excipient.

**Brief Description of Drawings**

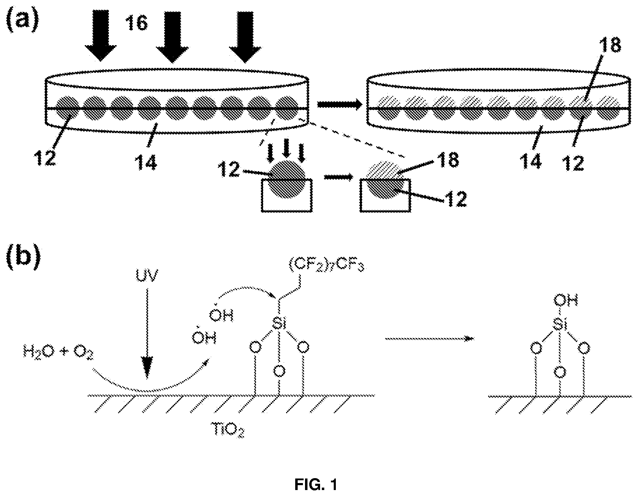

**Fig. 1** Depicts: (a) schematic representation for the preparation of Janus particles *via* UV irradiation of the particles assembled at the air-water interface; and (b) proposed mechanism for the degradation of FAS on $TiO_2$ surface induced by UV irradiation. Hydroxyl radicals generated by $TiO_2$ attack and cause cleavage of the alkyl chain.

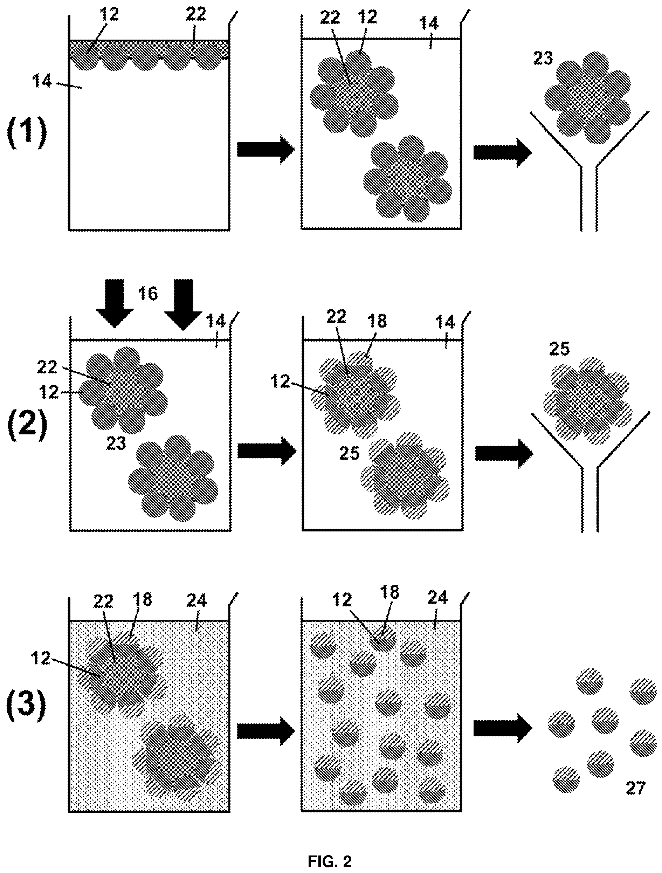

**Fig. 2** Depicts the schematic representation for the preparation of Janus particles *via* UV irradiation of the particles assembled in a Pickering emulsion in the presence of paraffin wax.

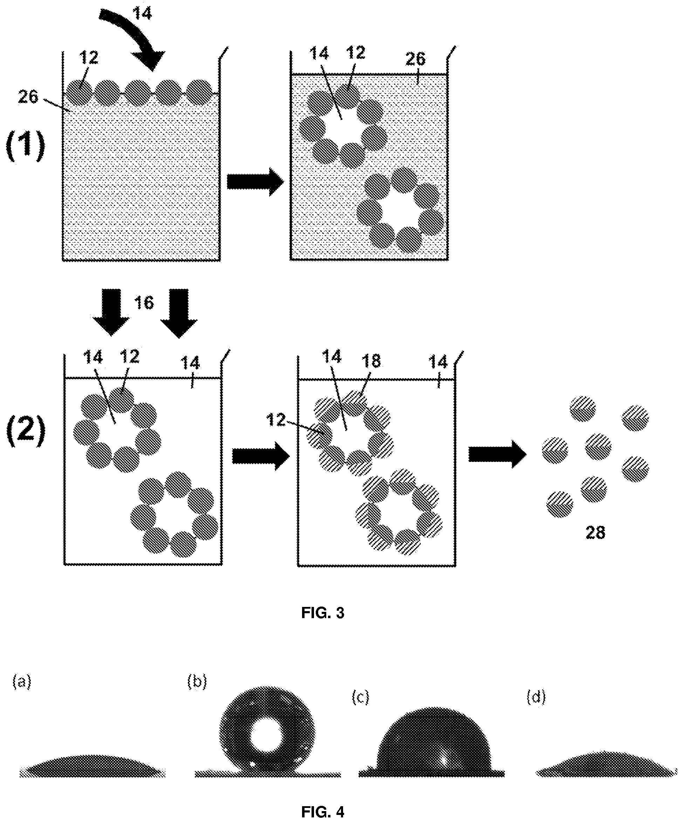

**Fig. 3** Depicts the schematic representation for the preparation of Janus particles *via* UV irradiation of the particles assembled in a water-in-oil Pickering emulsion.

**Fig. 4** Depicts the initial static contact angle measurements on powder films prepared using the FAS-$TiO_2$ of Example 1 (untreated and UV treated): (a) a water droplet on an anatase $TiO_2$ powder film; (b) a water droplet on an FAS-$TiO_2$ powder film (without UV irradiation); (c) a water droplet on an FAS-$TiO_2$ powder film irradiated by UV for 1 hr; and (d) for 2 hrs.

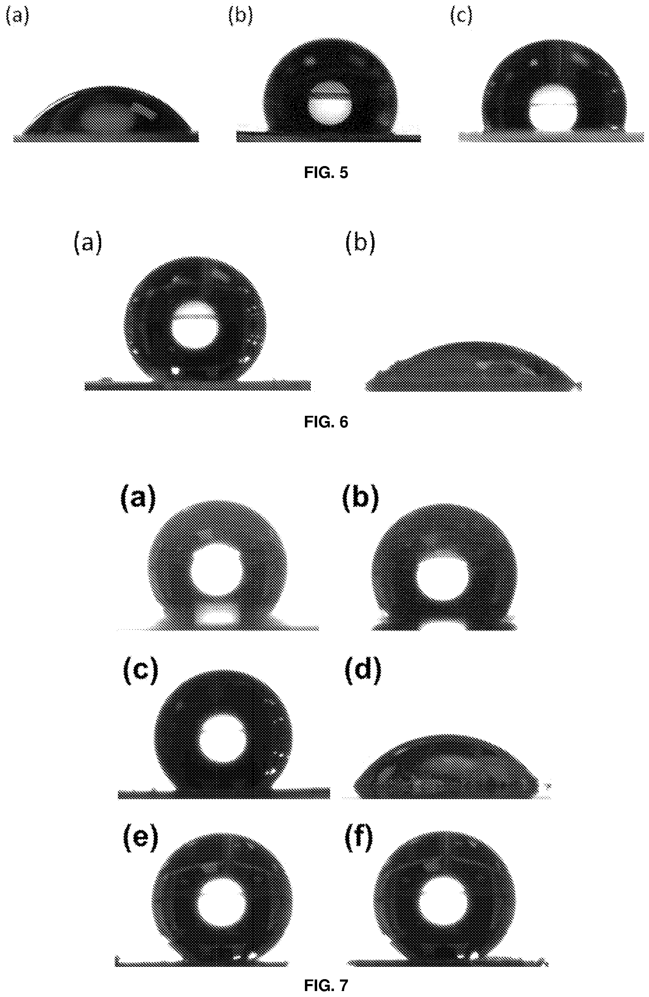

**Fig. 5** Depicts the initial static contact angle measurements on modified glass slides of Example 1 (untreated and UV treated): (a) a water droplet on a glass slide; (b) a water droplet on an FAS-modified glass slide; and (c) a water droplet on an FAS-modified glass slide irradiated by UV for 2 hrs.

**Fig. 6** Depicts the initial static contact angle measurements on powder films prepared using the HDTMS-$TiO_2$ of Example 1 (untreated and UV-treated): (a) a water droplet on an HDTMS-$TiO_2$ powder film; and (b) a water droplet on an HDTMS-$TiO_2$ powder film irradiated by UV for 1 hr.

**Fig. 7** Depicts the initial static contact angle measurements on powder films prepared using the FAS-modified silica-$TiO_2$, FAS-modified ZnO and FAS-modified $Cu_2O$ of Example 1, (untreated and UV-treated): (a) a water droplet on an FAS-modified silica-$TiO_2$ powder film; (b) a water droplet on an FAS-modified silica-$TiO_2$ powder film irradiated by UV for 2 hrs; (c) a water droplet on an FAS-modified ZnO powder film; (d) a water droplet on an FAS-modified ZnO powder film irradiated by UV for 3 hrs; (e) a water droplet on an FAS-modified $Cu_2O$ powder film; and (f) a water droplet on an FAS-modified $Cu_2O$ powder film irradiated by UV for 2 hrs.

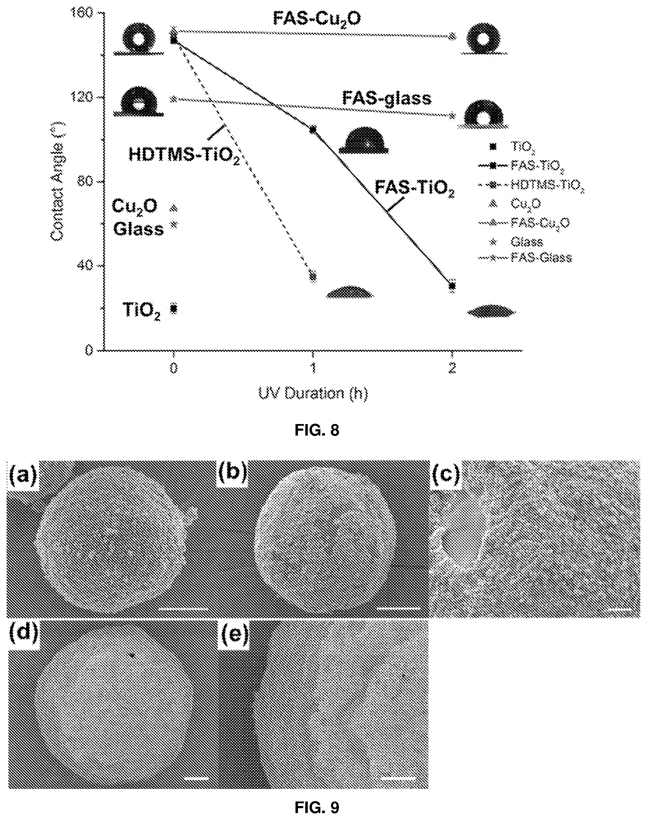

**Fig. 8** Depicts the subsequent measurements of the static contact angles of water droplets on powder films made from $TiO_2$ particles (squares), $Cu_2O$ particles (triangles), and glass substrates (stars) of Example 1, as a function of UV irradiation duration.

**Fig. 9** Depicts the FESEM images of wax droplet 25 (prepared in Example 2) stabilised by: (a) FAS-$TiO_2$ particles; (b) OTES-$TiO_2$ particles; and (d) FAS-silica $TiO_2$; (c) shows a close-up view of OTES-$TiO_2$ particles assembled over the surface of a wax droplet in (b); and (e) shows a close-up view of FAS-silica $TiO_2$ particles assembled over the surface of a wax droplet in (d). Scale bar represents 10 $\mu$m.

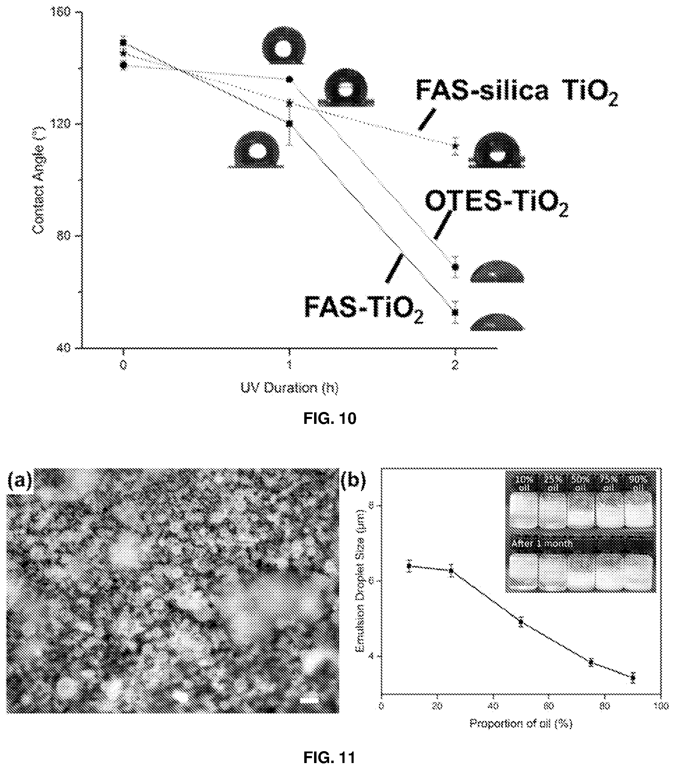

**Fig. 10** Depicts the static contact angles of water droplets on powder films made from FAS-$TiO_2$ particles (squares), OTES-$TiO_2$ particles (circles) and FAS-silica $TiO_2$ particles (stars) of Example 2, as a function of UV irradiation duration.

**Fig. 11** Depicts: (a) dark-field optical images of the Pickering water-in-oil emulsions (of Example 3) stabilised by FAS-$TiO_2$ particles. Scale bar represents 10 $\mu$m; and (b) a plot showing effect of silicone oil proportion on resulting sizes of emulsion droplets stabilised by FAS-TiO$_2$ particles. Inset shows the appearance of fresh emulsions (top), and emulsions aged for 1 month (bottom).

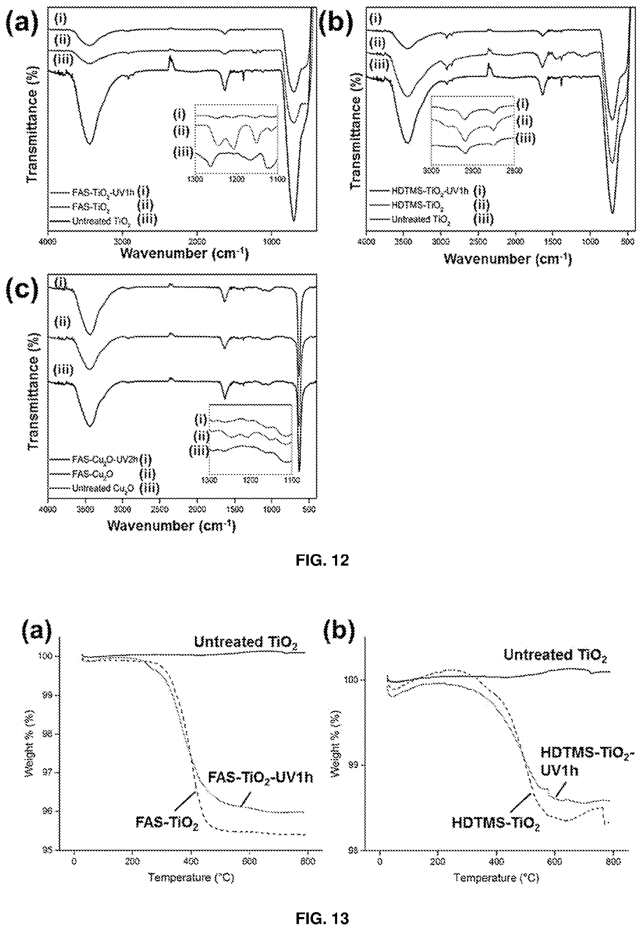

**Fig. 12** Depicts the FTIR spectra of Janus particles of Example 1: (a) FAS-TiO$_2$-UV1h in comparison with FAS-TiO$_2$ and untreated TiO$_2$; (b) HDTMS-TiO$_2$-UV1h in comparison with HDTMS-TiO$_2$ and untreated TiO$_2$; and (c) FAS-Cu$_2$O-UV2h in comparison with FAS-Cu$_2$O and untreated Cu$_2$O. Insets show the zoomed-in region of relevant FAS or HDTMS peaks.

**Fig. 13** Depicts plots of weight loss as a function of temperature for the initial TGA of the Janus particles of Example 1: (a) untreated anatase TiO$_2$, FAS-treated anatase TiO$_2$ and FAS-treated anatase TiO$_2$ irradiated by UV for 1 hr; and (b) untreated anatase TiO$_2$, HDTMS-treated anatase TiO$_2$ and HDTMS-treated anatase TiO$_2$ irradiated by UV for 1 hr.

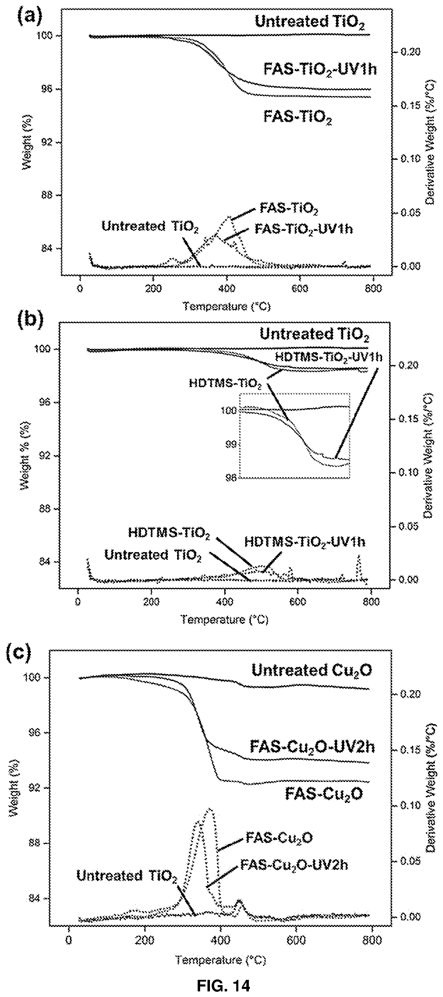

**Fig. 14** Depicts subsequent TGA analysis of the Janus particles of Example 1: (a) TGA curves (solid) showing weight loss as a function of temperature for untreated TiO$_2$, FAS-TiO$_2$, and FAS-TiO$_2$-UV1h particles; (b) TGA results of untreated TiO$_2$, HDTMS-TiO$_2$, and HDTMS-TiO$_2$-UV1h particles; and (c) TGA results for untreated Cu$_2$O, FAS-Cu$_2$O, and FAS-Cu$_2$O-UV2h particles. The corresponding DTG (dotted) curves for each set of particles are also shown.

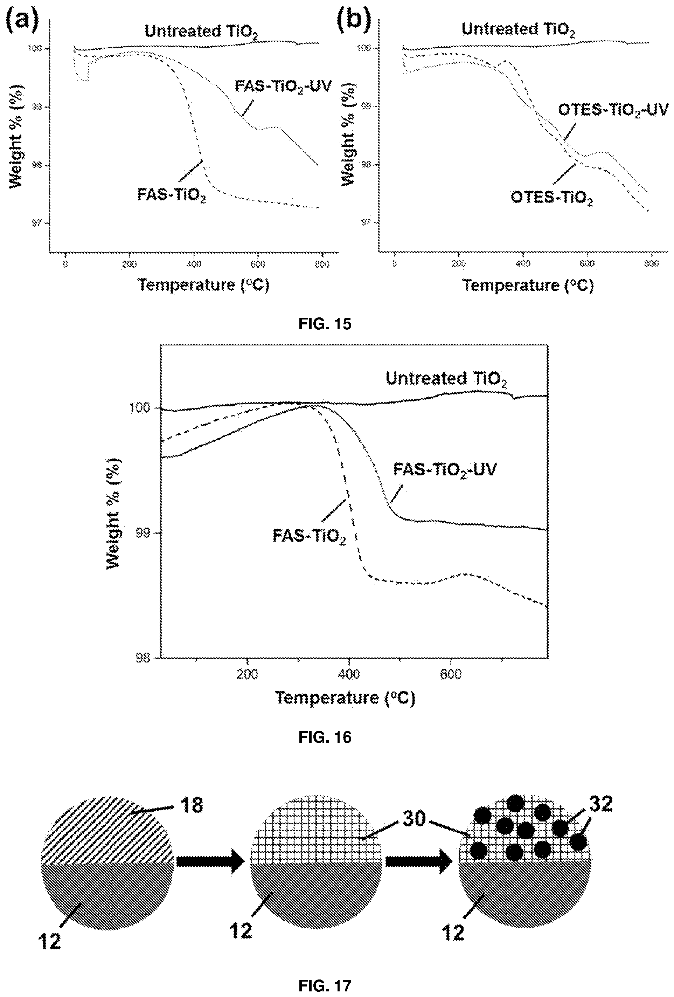

**Fig. 15** Depicts the TGA analysis of the Janus particles of Example 2: (a) plots of weight loss as a function of temperature of untreated anatase TiO$_2$, FAS-TiO$_2$ and FAS-TiO$_2$ irradiated by UV for 2 hrs; and (b) plots of weight loss as a function of temperature of untreated anatase TiO$_2$, OTES-TiO$_2$ and OTES-TiO$_2$ irradiated by UV for 2 hrs.

**Fig. 16** Depicts the TGA analysis of the Janus particles of Example 3: plots of weight percent as a function of temperature for the TGA of untreated TiO$_2$, FAS-TiO$_2$ and FAS-TiO$_2$-UV.

**Fig. 17** Depicts a schematic for the modification of the UV-irradiated surface **18** of the Janus particles of the current invention with silane **30,** followed by labelling with gold nanoparticles **32.**

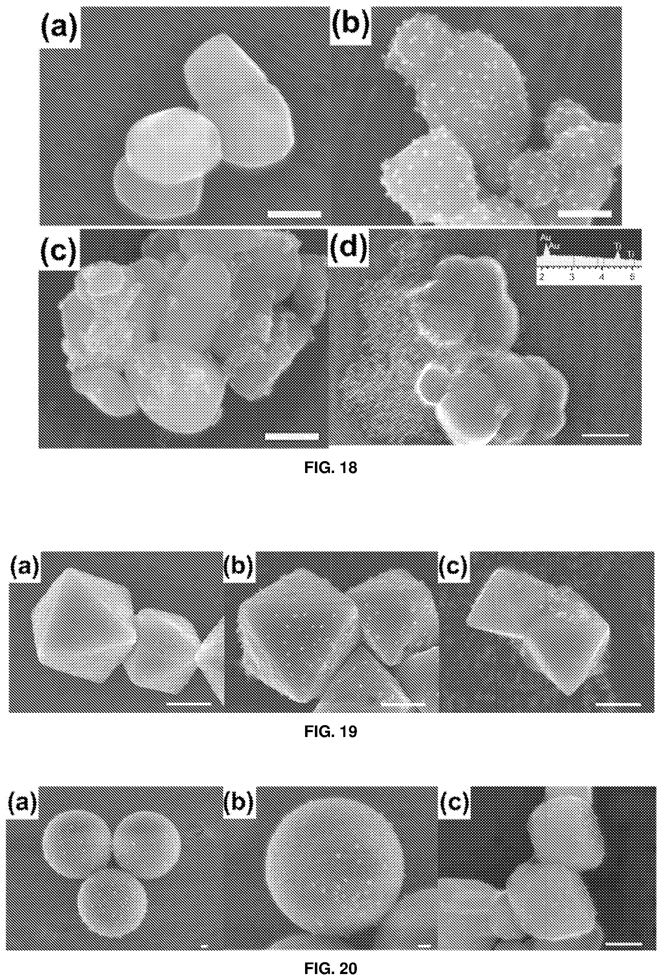

**Fig. 18** FESEM images of various TiO$_2$ particles (of Example 1) after labelling with 10 nm gold nanoparticles: (a) FAS-TiO$_2$; (b) untreated TiO$_2$; and (c) FAS-TiO$_2$-UV1h. The presence of the gold nanoparticles indicates the extent of irradiated surface over the TiO$_2$ particles. Fig. 18d shows the FAS-TiO$_2$-UV1h particles labelled with 10 nm gold NP, with excess gold NP on substrate. Inset shows the elemental peaks of gold and titanium present in the sample as analyzed by EDS. Scale bar represents 100 nm.

**Fig. 19** Depicts the FESEM images of various Cu$_2$O particles (of Example 1) after labelling with 30 nm gold nanoparticles: (a) FAS-Cu$_2$O; (b) untreated Cu$_2$O; and (c) FAS-Cu$_2$O-UV2h. The presence of the gold nanoparticles indicates the extent of irradiated surface over the Cu$_2$O particles. Scale bar represents 500 nm.

**Fig. 20** Depicts the FESEM images of various particles of Example 1: (a) silica-TiO2 particles irradiated by UV for 2 hrs, and treated with APTES and 30 nm gold nanoparticles; a close-up view of the treated silica-TiO2 particle; (c) ZnO particles irradiated by UV for 3 hrs, and treated with APTES and 10 nm gold nanoparticles. Scale bar represents 100 nm.

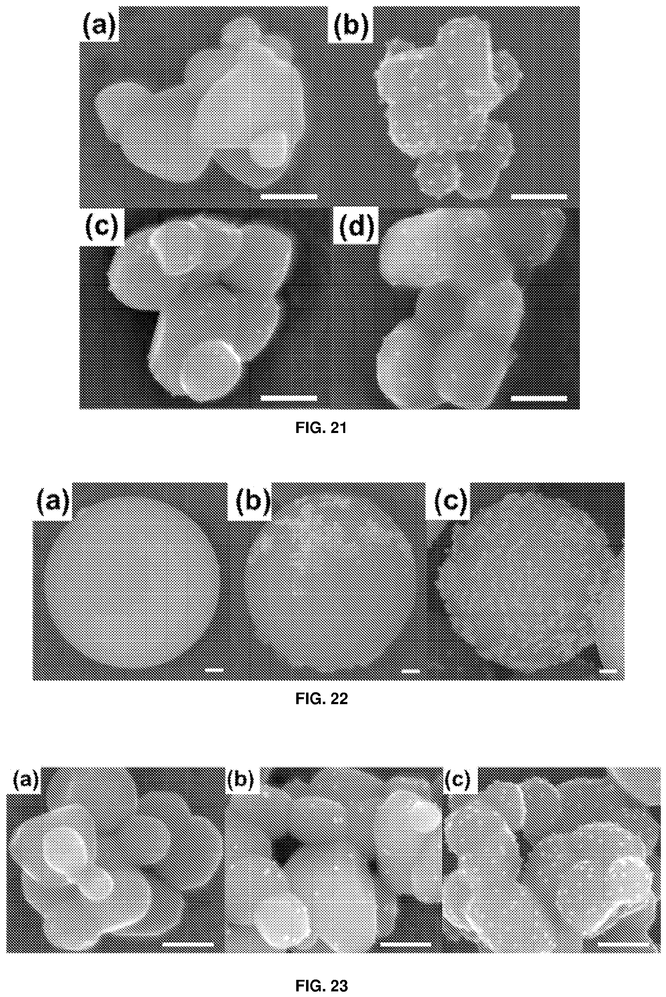

**Fig. 21** Depicts the FESEM images of various particles of Example 2 treated with APTES and 10 nm gold NP: (a) FAS-TiO$_2$ particles; (b) APTES-TiO$_2$ particles; (c) FAS-TiO$_2$ particles irradiated with UV for 1 hr; and (d) OTES-TiO$_2$ particles irradiated with UV for 1 hr. Scale bar represents 100 nm.

**Fig. 22** Depicts the FESEM images of various particles of Example 2 treated with APTES and 30 nm gold NP: (a) FAS-silica TiO$_2$ particles; (b) FAS-silica TiO$_2$ particles irradiated with UV for 1 hr; (c) APTES-silica TiO$_2$ particles. Scale bar represents 100 nm.

**Fig. 23** Depicts the FESEM images of various particles of Example 3 functionalised with APTES and 10 nm gold NP: (a) FAS-TiO$_2$; (b) FAS-TiO$_2$-UV; and (c) untreated TiO$_2$ particles. Scale bar represents 100 nm.

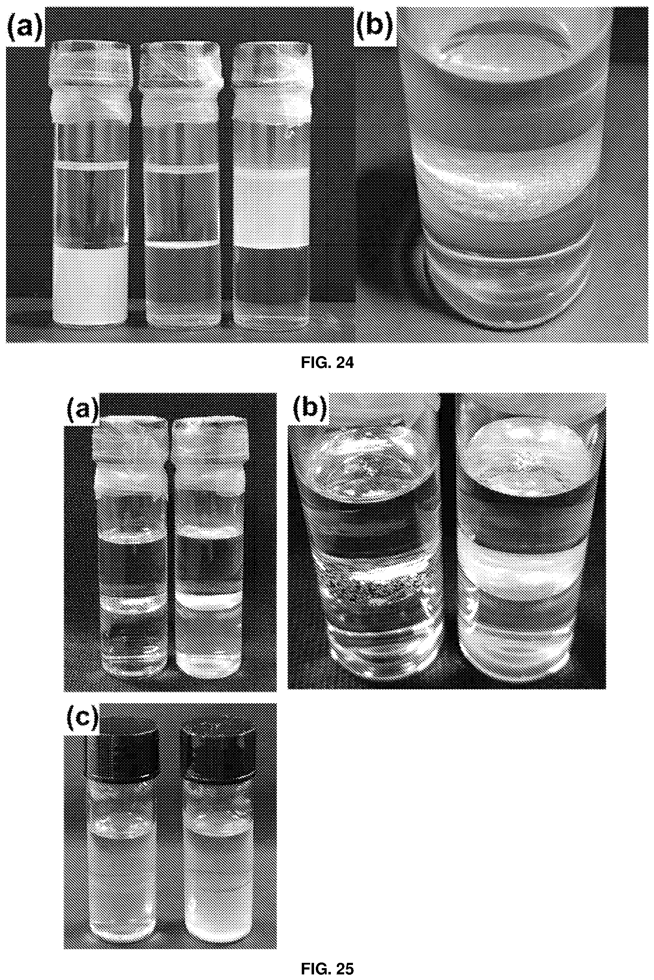

**Fig. 24** Depicts the partitioning of various particles in a toluene/water system following ultrasonic disruption: (a) TiO$_2$ particles (left) dispersed in the water phase, HDTMS-TiO$_2$-UV1h particles (center) remained at the toluene/water interface, and HDTMS-TiO$_2$ (right) particles dispersed in the toluene phase; and (b) close-up view of HDTMS-TiO$_2$-UV1h particles at the toluene/water interface.

**Fig. 25** Depicts different particles assembled at the toluene/water interface: (a) FAS-TiO$_2$ (left) and FAS-TiO$_2$-UV1h (right); (b) a close-up view of (a); and (c) FAS-Cu$_2$O (left) and FAS-Cu$_2$O-UV2h (right).

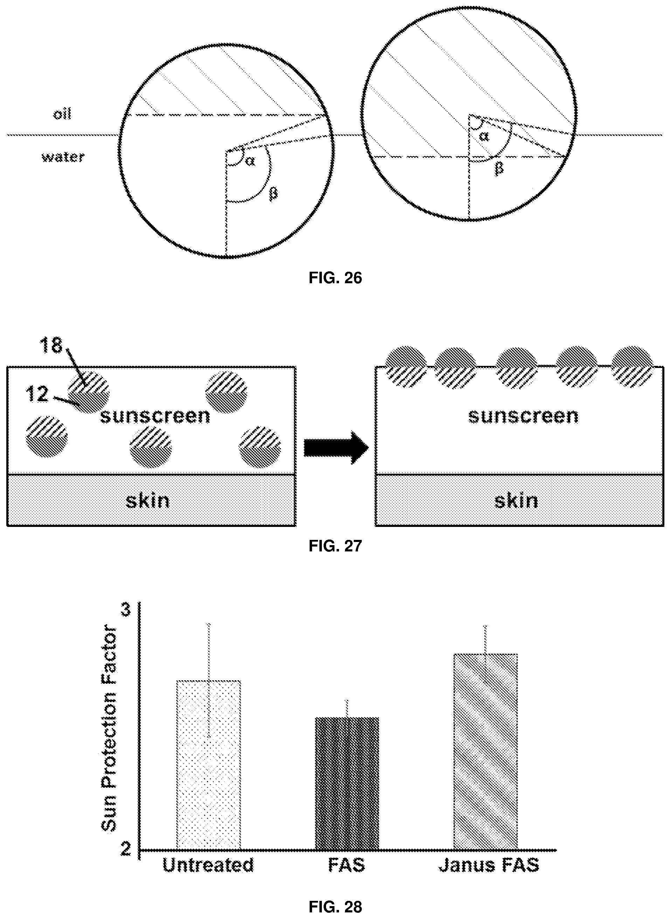

**Fig. 26** Depicts the geometry of Janus particles of varying wettabilities assembled at an oil/water interface: (a) denotes the position of the surface boundary between hydrophobic and hydrophilic regions; (b) parametrises the immersion depth of the particle. Shaded portions refer to hydrophobic regions.

**Fig. 27** Depicts the self-assembly of the Janus particles of the current invention containing amphiphilic **(18)** and amphiphobic **(12)** domains in a sunscreen formulation.

**Fig. 28** Depicts the Sun Protection Factor (SPF) values based on initial studies for sunscreens made from untreated, fully FAS treated, and irradiated FAS (Janus) particles, with 5 wt.% particle loading.

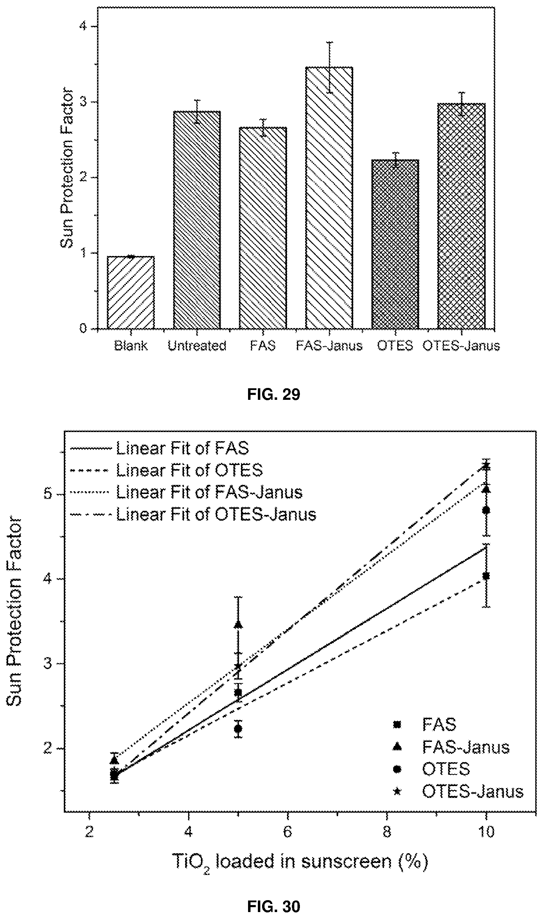

**Fig. 29** Depicts the SPF values of various sunscreen formulations using different types of TiO$_2$ particles (treated or untreated), with 5 wt.% particle loading.

**Fig. 30** Depicts the SPF values of various sunscreen formulations using different percent loading of TiO$_2$ particles: FAS (squares), FAS-Janus (triangles), OTES (circles), and OTES-Janus (stars). Linear fits of the data points for each sunscreen are also shown.

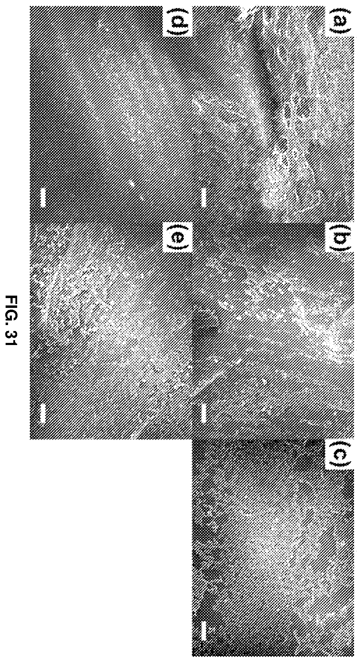

**Fig. 31** Depicts the appearance of VITROSKIN surface following application of different sunscreens made from: (a) untreated particles; (b) FAS particles; (c) FAS-Janus particles; (d) OTES particles; and (e) OTES-Janus particles, at 5 wt.% particle loading. Scale bar represents 10 $\mu$m.

## Description

It has been surprisingly found that Janus particles can be formed easily and cheaply, with consistent bulk properties that make them suitable for use in a range of different applications. For example, in sun lotions or in other cosmetic formulations.

Thus, disclosed herein is a Janus particle comprising:

a core inorganic photocatalytic particle having a surface with a first region and a second region and an average diameter of from 50 nm to 10 $\mu$m; and
a low surface energy organic coating, which is a C$_8$ to C$_{26}$ alkylsilane or a fluoroalkylsilane coating that is susceptible to photo-degradation, which coating is covalently bound to the surface of the core inorganic photocatalytic particle where present, wherein:

the low surface energy organic coating fully coats the entire surface of the first region and the second region is coated by a residue formed by photodegradation of the coating, such that the first region displays hydrophobic or hydrophobic and oleophobic properties and the second region displays amphiphilic properties.
When used herein, a "Janus particle" refers to a particle that displays asymmetric surface properties. For example, the Janus particle may contain two regions displaying different surface properties, where the two regions may be of balanced surface area or one may be bigger than the other. In such particles, the surface of one region may display hydrophobic/oleophilic or amphiphobic properties, while the other region displays amphiphilic properties.

As will be appreciated, the first and second regions referred to above may have any suitable combination of surface areas - provided that they encompass the entire surface area of the Janus particle. For example, the first region may form from 30 to 70% of the surface area of the Janus particle, such that the second region forms from 70 to 30% of the surface area of the Janus particle. In additional or alternative embodiments, the first region may form from 40 to 60% of the surface area of the Janus particle, such that the second region forms from 60 to 40% of the surface area of the Janus particle. In particular embodiments that may be disclosed herein, the first and second regions may each form about 50% of the surface area of the Janus particle.

In embodiments herein, the word "comprising" may be interpreted as requiring the features mentioned, but not limiting the presence of other features. Alternatively, the word "comprising" may also relate to the situation where only the components/features listed are intended to be present (e.g. the word "comprising" may be replaced by the phrases "consists of" or "consists essentially of"). It is explicitly contemplated that both the broader and narrower interpretations can be applied to all aspects and embodiments of the present invention. In other words, the word "comprising" and synonyms thereof may be replaced by the phrase "consisting of" or the phrase "consists essentially of" or synonyms thereof and *vice versa*.

When used herein, the term "inorganic photocatalytic particle" refers to an inorganic material in particulate form that exhibits photocatalytic activity upon exposure to light having an energy higher than a predetermined band gap. Any suitable inorganic material (or combination thereof) may be used in the current invention. Examples of suitable materials that may be used as the core particle of the Janus particle include one or more of $CeO_2$, $SnO_2$, $Nb_2O_5$, $WO_3$, $Fe_2O_3$, $Ta_2O_3$, $CuO$, $NiO$, $Cr_2O_3$, $RuO_2$, $TiO_2$, $ZnO$, $Cu_2O$.

In additional or alternate embodiments, the core particle of the Janus particle may be formed from a hybrid core-shell particle. In principle, when a hybrid core-shell particle is used as the core structure, any suitable solid material may be used as the core portion, as it is intended to act as a physical carrier of the photocatalytic shell. For example, the core portion may be selected from one or more of silica, a polymer, a ceramic, a metal and an alloy (e.g. the core portion may be selected from one or more of silica, a ceramic, a metal and an alloy). The shell of the hybrid core-shell particle may be selected from one or more of $CeO_2$, $SnO_2$, $Nb_2O_5$, $WO_3$, $Fe_2O_3$, $Ta_2O_3$, $CuO$, $NiO$, $Cr_2O_3$, $RuO_2$, $TiO_2$, $ZnO$, and $Cu_2O$. In more specific embodiments that may be mentioned herein, the hybrid core-shell particle may be silica surrounded by a shell comprising one or more of $CeO_2$, $SnO_2$, $Nb_2O_5$, $WO_3$, $Fe_2O_3$, $Ta_2O_3$, $CuO$, $NiO$, $Cr_2O_3$, $RuO_2$, $TiO_2$, $ZnO$, and $Cu_2O$. Unless otherwise stated, the metal oxides mentioned herein may be present in a single Janus particle (i.e. as an alloy) or in separate Janus particles (e.g. some Janus particles containing $CuO$ and some containing $NiO$), whether as the whole of the particle or as the shell surrounding a silica core. In particular embodiments of the invention that may be disclosed herein, the core inorganic photocatalytic material may be selected from one or more of $TiO_2$, $ZnO$, and $Cu_2O$. For example, the core inorganic photocatalytic material may be $TiO_2$.

Without wishing to be bound by theory, when the core portion of the hybrid core-shell particle is formed from a polymer, it is believed that the shell of the hybrid core-shell particle will shield the polymer in the core portion of said particle from the effects of UV light, such that the polymer will not be affected, at least with regard to the timescales contemplated with regard to the method of manufacture and resulting use of said materials herein.

The core inorganic photocatalytic particle may have any suitable size. For example, the core inorganic photocatalytic particle may have an average diameter of from 50 nm to 10 $\mu$m, such as from 75 nm to 5 $\mu$m.

When used herein, the term "low surface energy organic coating" is intended to refer to a material that provides a high contact angle >90° when a surface coated in said material is contacted by water. The low surface energy material forms a covalent bond with the surface of the core inorganic photocatalytic particle in the current invention, such that the low surface energy material becomes covalently bound to the surface of the core inorganic photocatalytic particle. For example, suitable compounds capable of forming a covalent bond with the core inorganic photocatalytic particle include, but are not limited to $C_8$ to $C_{26}$ alkyltrialkoxysilanes (e.g. octyltriethoxysilane and/or hexadecyltrimethoxysilane) and fluoroalkyltrialkoxysilanes (e.g. 1 H,1 H,2H,2H-perfluoro-decyltriethoxysilane). The resulting low surface energy organic coating is formed by $C_8$ to $C_{26}$ alkylsilanes (e.g. octylsilane and/or hexadecylsilane) or fluoroalkylsilanes covalently bound to the surface of the core inorganic photocatalytic particles. As will be appreciated, one or more of the low surface energy organic coating compounds described hereinbefore may be used to form the low surface energy organic coating. In particular embodiments of the invention that may be mentioned herein the low surface energy organic coating may be a $C_6$ to $C_{26}$ fluoroalkylsilane, such as 1H,1H,2H,2H-perfluorodecylsilane, covalently bound to the surface of the core inorganic photocatalytic particle.

When used herein, a "degraded low surface energy organic coating" refers to a region of a Janus particle where a residue of the low surface energy organic coating remains on the surface of the Janus particle in the first region. Such residues remain on the surface of the core inorganic photocatalytic particle when the low surface energy organic coating is subjected to photodegradation, but not all of the covalently bound material is removed. Fig. 1b shows an example of this where an alkylsilane or a fluoroalkylsilane is subjected to a photodegradation reaction, but leaves a silanol residue on the surface of the inorganic particle.

When used herein, "hydrophobic" refers to the property of a material to repel water (i.e. a material that displays a high contact angle (e.g. >90°) when water is dropped onto a surface composed of said material). When used herein, "oleophilic" refers to the property of a material to display a low contact angle (e.g. <90°) when an oil is dropped onto a surface composed of said material. In certain embodiments mentioned herein the terms "hydrophobic" and "oloephililc" may be used interchangeably. Examples of hydrophobic/oleophilic materials that may be mentioned herein include, but are not limited to surfaces formed by covalently bonding octylsilane or hexadecylsilane to the surface of an inorganic particle of the type mentioned hereinbefore, such that these materials become covalently bound to the surface of the core inorganic photocatalytic particle.

When used herein, "amphiphobic" refers to the property of a material to display hydrophobic and oleophobic properties (i.e. high contact angles (e.g. >90°) when water or an oil (e.g. silicone oil) is dropped onto its surface. Examples of amphiphobic materials that may be mentioned herein include, but are not limited to surfaces formed by covalently bonding fluoroalkylsilanes (e.g. 1H,1H,2H,2H-perfluorodecylsilane) to the surface of an inorganic particle of the type mentioned hereinbefore, such that these fluoroalkylsilanes become covalently bound to the surface of the core inorganic photocatalytic particle.

When used herein, "amphiphilic" refers to the property of a material to display hydrophilic and oleophilic properties (i.e. low contact angles (e.g. <90°) when water or an oil (i.e. silicone oil) is dropped onto its surface. Examples of amphiphilic surfaces may refer to the bare inorganic photocatalytic particle surface or of said surface that is coated by the low surface energy organic coating that has been partly or fully degraded.

It will be appreciated that the above contact angles refer to the inherent properties of the individual materials, or specific combination of materials (such as the combination of covalently bound low surface energy molecules covering the surface of the core inorganic photocatalytic particle) used to form the first and second regions of the Janus particles disclosed herein. As such, it is to be expected that the overall properties of the Janus particles differ from the materials used to form the first and second regions on the surface of the Janus particle, as the Janus particle contains both regions. Suitable water contact angles for the Janus particles of the current invention, when presented in the form of a compact film as described in the examples, may be from 30 to 140°. For example, the water contact angle may be from 50 to 120°, such as from 80 to 110°.

For the avoidance of doubt, when a number of related numerical ranges are disclosed herein in relation to a specific feature of the invention, it is explicitly intended that the upper and lower limits of said ranges may be combined in any manner possible. For example, for the water contact angles disclosed herein, the following ranges are explicitly intended to be disclosed:

30 to 50°, 30 to 80°, 30 to 110°, 30 to 120°, 30 to 140°;
50 to 80°, 50 to 110°, 50 to 120°, 50 to 140°;
80 to 110°, 80 to 120°, 80 to 140°;
110 to 120°, 110 to 140°; and
120 to 140°.

The Janus particles disclosed herein may be made by any suitable method. Thus, there is also disclosed a method of manufacturing a Janus particle, the method comprising the steps of:

(a) providing an aqueous dispersion of precursor Janus particles comprising a core inorganic photocatalytic particle, the core inorganic photocatalytic particle having an average diameter of from 50 nm to 10 $\mu$m, wherein the core inorganic photocatalytic particle has a surface that is fully coated by a $C_8$ to $C_{26}$ alkylsilane or a fluoroalkylsilane coating that is susceptible to photo-degradation, which coating is covalently bound to the surface of the core inorganic photocatalytic particle; and
(b) subjecting the aqueous dispersion to irradiation by ultra-violet light for a period of time to provide Janus particles comprising:

the core inorganic photocatalytic particle having a surface with a first region and a second region; and
a $C_8$ to $C_{26}$ alkylsilane or a fluoroalkylsilane coating that is susceptible to photo-degradation, which coating is covalently bound to the surface of the core inorganic photocatalytic particle where present, wherein:

the coating fully coats the entire surface of the first region and the second region is coated by a residue formed by photo-degradation of the coating, such that;
the first region displays hydrophobic or hydrophobic and oleophobic properties; and the second region displays amphiphilic properties, wherein
the period of time is from 30 minutes to 2 hours;
the ultra-violet light is provided at a wavelength of from 100 to 400 nm; and

the ultra-violet light is provided at an irradiance value of from 50 to 150 mW/cm$^2$.

Unless otherwise specified below, where the materials and terms used above are identical to those used in relation to the Janus particles *per se,* then the same definitions apply. The core inorganic photocatalytic particles are the same as those defined hereinbefore.

The general process described above covers three possible processes that may be used to manufacture the Janus particles of the current invention. The first process simply involves the steps set out above, while the second process further partially coats the Janus precursor in a wax that covers from 30 to 70%, such as from 60 to 40% (e.g. 50%) of the surface area of the precursor Janus particle before step (b) above is conducted. The third process presents the precursor Janus particles as part of a Pickering emulsion droplet, obtained from a water-in-oil emulsion that is dispersed into water.

The precursor Janus particles for use in the first process referred to above may be prepared by reacting the core inorganic photocatalytic particles with any suitable material that can provide a low surface energy organic coating that is covalently bound to the surface of the core inorganic photocatalytic particles. For example, the core inorganic photocatalytic particles may be reacted with one or more of a $C_8$ to $C_{26}$ alkyltrialkoxysilane and a fluoroalkyltrialkoxysilane to provide the precursor Janus particles. In more particular embodiments, the core inorganic photocatalytic particles may be reacted with one or more of octyltriethoxysilane, hexadecyltrimethoxysilane, and 1H,1H,2H,2H-perfluoro-decyltriethoxysilane. Any suitable ratio of low surface energy organic coating to core inorganic photocatalytic particles may be used in this process. For example, the volume:volume ratio of low surface energy organic coating to core inorganic photocatalytic particles may be from 1:1 to 1:100, such as from 1:10 to 1:75, such as from 1:20 to 1:50, such as about 1:40 (e.g. the volume to volume ratio of 1H,1H,2H,2H-perfluoro-decyltriethoxysilane to $TiO_2$ may be 1:40).

The reaction method to form the precursor Janus particles used directly in the first process may involve mixing a solution containing the core inorganic photocatalytic particles in a solvent with a solution containing the desired precursor of the low surface energy organic coating in a solvent. The resulting reaction solution may be mixed at room temperature or at elevated temperature for a suitable period of time until the core inorganic photocatalytic particles are fully covered by the low surface energy organic coating. Further details of how such reactions may be conducted (and the associated purification steps) are provided in the experimental section below.

It is noted that these precursor Janus particles described above are also used as a starting material for the second and third processes described in more detail below.

In the first of the processes described above, the process simply involves dispersing the precursor Janus particles described above directly into a dish of water, which is then subjected to UV light irradiation for a suitable period of time. This results in the direct formation of the Janus particles described hereinbefore and has the advantage of being a simple and straightforward process. It is noted that the precursor Janus particles float at the air-water interface and do not tend to significantly rotate, as evidenced by the differential properties of the Janus particles obtained in the experimental section below that make use of this method. Nevertheless, as the particles are able to rotate to a certain degree, it is noted that leaving the particles for an extended time under irradiation using the first process will remove the entire low surface energy organic coating. Further details of how the first process is conducted is provided in the examples section below.

For the second process, the precursor Janus particles prepared for direct use in the first process are partially covered in wax before they are added to a dish of water for the UV light irradiation step discussed above in the first process. This partial coating of wax may be achieved by dispersing the precursor Janus particles prepared for direct use in the first process in water held at an elevated temperature (i.e. a temperature that will melt the chosen wax) and then adding the chosen wax to the resulting mixture. Once the wax has melted, the mixture is subjected to vigorous mixing (e.g. from 10,000 to 20,000 rpm, such as 15,000 rpm) for a short period of time (e.g. from 1 to 5 minutes, such as 2 minutes) to provide a precursor Janus particle that is partially embedded in a wax droplet, leaving a portion of the low surface energy organic coating fully exposed. The resulting particles are then subjected to the same UV light irradiation process described above for the first process, but with stirring of the particles to ensure that the uncoated surface of the particles is fully degraded. Any suitable speed of stirring may be used in this process, such as (but not limited to) about 100 rpm. The wax may then be removed from the Janus particle by dissolution into a suitable organic solvent.

Any suitable wax may be used for the coating process described above. A suitable wax is one that protects the low surface energy organic coating of the precursor Janus particle that is covered by the wax from degradation by UV light during the irradiation step described herein. Thus, the second process has the advantage that if the particles are subjected to excessive irradiation, they will still provide Janus particles by virtue of the protective wax coating. The wax coating may cover from 30 to 70%, such as from 60 to 40% (e.g. 50%) of the surface area of the precursor Janus particle.

As indicated above, each wax droplet houses a number of partly-embedded precursor Janus particles. Ideally, the embedded precursor Janus particles form a monolayer on the surface of each wax droplet (which is ideally spherical or spheroidal). Whether such a monolayer is formed or not is influenced by the amount of low surface energy organic coating on the precursor Janus particles. A precursor Janus particle functionalised using a high concentration of low surface energy organic coating (e.g. a volume:volume ratio of low surface energy organic coating to core inorganic photocatalytic particles of about 1:4) is not optimal, as it will tend to agglomerate with other precursor Janus particles and inhibit the formation of spherical wax droplets with the desired monolayer of said precursor Janus particles. However, even when this occurs, the aggregated particles are still effectively masked by each other, and UV irradiation of such droplets should still produce Janus particles.

Further details of how the second process is conducted is provided in the examples section below.

For the third process, the precursor Janus particles prepared for direct use in the first process are used as Pickering emulsion stabilisers to form a water-in-oil emulsion. This may be achieved by first adding the precursor Janus particles that are used directly in the first process described above to an oil to form a mixture that is then subjected to vigorous mixing (e.g. from 10,000 to 20,000 rpm, such as 15,000 rpm), to which water is added in drop-wise fashion to form a water-in-oil mixture. Any suitable ratio of oil to water may be used (e.g. from 90:10 vol:vol oil to water to 50:50 vol:vol oil to water). Mixing may be continued for a period of time after all of the water has been added. The resulting emulsions provide droplets having a diameter of from 1 to 10 $\mu m$ (e.g. 3.44 $\mu m$), where the precursor Janus particles act to stabilise the Pickering emulsion. In certain embodiments of the invention that may be disclosed herein, the low surface energy organic coating that is covalently bound to the surface of the core inorganic photocatalytic particle may be a fluoroalkylsilane (e.g. 1H,1H,2H,2H-perfluorodecylsilane), as this provides a particle that is amphiphobic, thereby encouraging the particles to align at the interface between the oil and water, which may result in a more stable Pickering emulsion.

In order to form the desired Janus particles, the Pickering emulsion formed above may be dispersed into a dish of water and subjected to UV light irradiation and stirring as described in the second process above.

Further details of how the third process is conducted is provided in the examples section below.

In all of the processes described above, the UV light irradiation may last for any suitable period of time. For example, the irradiation may be for 1 hour. As will be appreciated, the amount of time allotted to the irradiation may vary depending on the irradiance value of the ultra-violet light. For example, the ultra-violet light may be provided at an irradiance value of from 70 to 140 mW/cm$^2$ (e.g. 72 mW/cm$^2$ or 136 mW/cm$^2$). When the irradiance value is low, the period of time may be higher and *vice versa.*

For the first process described above, it will be noted that the irradiation period of time should be sufficiently long to degrade a portion of the low surface energy organic coating, but not so long as to allow for complete degradation of the coating (i.e. the period of irradiation may be for 1 hour). This is because the particles are free to rotate in the aqueous dispersion. In contrast, the period/irradiance value of UV light irradiation may not be so important for the second and third processes. For example, in the second process, part of the precursor Janus particle is protected from photodegradation by the presence of the wax coating. In the third process, the formation of an emulsion may restrict the ability of the precursor Janus particles to rotate, thereby minimising over-exposure of the surface of the Janus particles even when the irradiation period is longer than would be optimal for the first process. Nevertheless, it would be expected that the UV light irradiation period and irradiance value should be optimised in an industrial process in order to minimise energy wastage.

The UV light used in the processes above may have a wavelength of from 300 to 395 nm, such as 365 nm.

The Janus particles disclosed herein may be particularly suitable for generating a self-assembled layer of particles for compositions that may be applied to an uneven surface, such as a skin. As such, the Janus particles may be particularly suitable for inclusion in cosmetic formulation, skin creams and sun lotions. Without wishing to be bound by theory, it is believed that the amphiphobic or hydrophobic/oleophilic face of the Janus particle is repelled by the skin and the rest of the formulation, so that the particles are driven upwards to the air-formulation interface, where they are held in place by the amphiphilic UV-treated face of the Janus particles, which serve to anchor the particles at the interface, consequently forming a desirable even layer of Janus particles at the air-formulation interface. As is well known, inorganic photocatalytic particles (e.g. TiO$_2$) are commonly used in sunscreens and cosmetics to provide a degree of protection from UV-light, but the amount of the material used tends to be high. Unlike conventional inorganic particles, Janus particles form a layer at the air-formulation interface. As such, an advantage associated with the Janus particles of the current invention is that they may be used to provide increased sun protection factors in suitable formulations or may be added in reduced quantities compared to conventional inorganic particles used for such purposes, while retaining an equivalent sun protection factor. Details of this advantage are discussed in more detail in the examples section below.

Thus, the invention also discloses a sunscreen formulation comprising Janus particles as described herein and a diluent, carrier or excipient. Any suitable formulation of sunscreen may be used, wherein the Janus particles may form from 1 to 10 wt% of the formulation. Examples of suitable sunscreen formulations that may be adapted for use with the current invention include those disclosed in Wiechers, S. et al., Cosmetics & Toiletries 2013, 128 (5), 2-6.

Further aspects and embodiments of the invention are discussed in more detail below with reference to the examples.

**Examples**

Materials and instruments

Anatase TiO$_2$ (1171 titanium dioxide E 171) was obtained from KRONOS. Methanol was obtained from Fisher Chemical. Absolute ethanol and sodium hydroxide were obtained from Merck. 1H,1H,2H,2H-perfluorodecyltriethoxysilane (FAS), hexadecyltrimethoxysilane (HDTMS), (3-aminopropyl)triethoxysilane 99% (APTES), toluene, 10 nm and 30 nm gold nanoparticles suspended in citrate buffer, copper (II) chloride dihydrate, polyvinylpyrrolidone (PVP, MW=40,000), L-ascorbic acid, and potassium bromide were obtained from Sigma Aldrich. Water used was purified water (Millipore, 18.2 MΩ·cm at 25 °C).

*Contact angle measurement*

Static contact angle measurements were taken using the contact angle goniometer OCA 20 (Dataphysics, Germany) and SCA 20 was used for analysis of the droplets. Approximately 2 mg of particles were placed on a glass slide, packed into a solid mound, and another glass slide was placed on top. The particles were manually hand-pressed into a consolidated film. All measurements were carried out at 25 °C in air, using 4 μL water as the test solvent. A minimum of three measurements was taken and their mean and standard deviation were reported.

*Fourier-transform infrared spectroscopy (FTIR)*

Particles were ground and mixed with potassium bromide using a mortar and pestle, then pressed under a 10 ton force into a pellet measuring 13 mm in diameter. The pellet was analysed by FTIR (Frontier, Perkin Elmer) from 4000 $cm^{-1}$ to 400 $cm^{-1}$ with a resolution of 4 $cm^{-1}$ with 32 scans.

*Thermogravimetric analysis (TGA)*

TGA was performed on the particles using SDT Q600 (TA Instruments). Particles were loaded in an alumina crucible. Under a nitrogen flow of 100 mL/min, the particles were subjected to a temperature ramp of 20 °C/min from 25 °C to 800 °C. The data was analysed using TA Universal Analysis.

*Field-emission scanning electron microscopy (FESEM)*

Particles were drop cast from their suspending solution on an aluminum foil substrate. They were observed under FESEM (JEOL JSM-7600F) operating at an accelerating voltage of 10 keV in secondary electron imaging mode. Energy-dispersive X-ray spectroscopy (EDS) was performed at 20 keV using X-Max (Oxford Instruments), and the EDS data was analysed by INCA.

General methods

*Surface functionalisation of $TiO_2$, $Cu_2O$, ZnO, silica-$TiO_2$ particles, glass substrates*

Typically, $TiO_2$ particles (with average diameter of 155 nm) was dispersed in methanol with a concentration of 2 g/L. Separately, FAS was dissolved dropwise in methanol, followed by a slow addition of water into the solution to hydrolyse the FAS. The resulting 1% v/v FAS solution was stirred for an hour to complete the hydrolysis process. For example, the 1% v/v FAS solution can be first prepared by dissolving 0.5 mL of FAS in 48 mL of methanol, followed by the dropwise addition of 1.5 mL of water. To functionalise the metal oxide particles, the FAS solution was added dropwise to the particles in a pre-determined volume ratio. For example, the FAS solution was then added to the $TiO_2$ solution in a 1:40 (FAS:$TiO_2$) volume ratio, making a 60 mL solution in total. The resulting FAS-metal oxide dispersion was stirred for 1 hr, and then heated at 100 °C for 1 hr. The reaction scheme of this functionalisation is similar to the reported alkylsilane modification (G. L. Witucki, J. Coat. Technol. 1993, 65, 57). The as-synthesised particles were recovered by centrifugation and washed twice with methanol to remove any excess FAS, then left to dry overnight at room temperature.

The same procedure was used for functionalisation of: $TiO_2$ particles with HDTMS; and $Cu_2O$, ZnO and silica-$TiO_2$ particles ($TiO_2$ particles with a silica core) with FAS. All the particles ($TiO_2$, ZnO, $Cu_2O$, silica-$TiO_2$) were first prepared in methanol at a concentration of 2 g/L, and 1% v/v FAS and HDTMS solutions were used. Different volume ratios of the particle suspension to the FAS/HDTMS solution were used for different particles. For example, a volume ratio of 1:40 was used for HDTMS:$TiO_2$, 1:60 for FAS:ZnO, 1:100 for FAS:$Cu_2O$, and 1:125 for FAS:silica-$TiO_2$.

Glass substrates were cleaned with water and dried prior to FAS functionalisation. The substrates were submerged in methanol, followed by the dropwise addition of FAS solution.

The resulting solution was stirred for 1 hr. The glass substrates were then removed from the solution and heated at 100 °C for 1 hr, before they were washed with methanol and dried.

*Synthesis of octahedral cuprous oxide particles*

Octahedral copper (I) oxide particles were synthesised following the procedure from Zhang *et* al. with slight modifications (D.-F. Zhang, et al., J. Mater. Chem. 2009, 19, 5220-5225). Briefly, at 55 °C, 4.44 g of PVP was dissolved in 0.01 M aqueous solution of $CuCl_2$. 2 M NaOH aqueous solution was added dropwise and stirred for 30 min, followed by the dropwise addition of 0.6 M ascorbic acid aqueous solution and stirred for 3 hrs. The recovered particles were washed with water and ethanol.

**Example 1. Preparation and characterisation of Janus particles *via* UV irradiation of the particles assembled at the air-water interface**

The preparation of the Janus particles of the current invention was carried out as shown in Fig. 1a, using the surface-functionalised particles prepared in accordance to the general method described above.

In a petri dish containing water **14,** the functionalised particles **12** were scattered over the surface of water and magnetically stirred for 30 min to disperse them across the air-water interface. The dish was placed directly under a Xe light source (Newport), at an irradiance at approximately 132 mW/cm$^2$. Irradiation (16) of the particles was performed for 1- or 2-hr durations, producing Janus particles of varying wettability which is dependent on the irradiation duration. The surface exposed to the UV irradiation underwent photocatalytic decomposition of the hydrophobic silanes, resulting in the formation of hydrophilic surfaces 18. The resulting particles are denoted as FAS-TiO$_2$-UV1h, FAS-TiO$_2$-UV2h, HDTMS-TiO$_2$-UV1h, and FAS-Cu$_2$O-UV2h accordingly. FAS-functionalised glass substrates were placed directly under the light source for 2 hrs, and denoted as FAS-glass-UV2h.

The same procedure was also used on HDTMS-functionalised TiO$_2$ particles, and for FAS-functionalised zinc oxide (ZnO) and 1 $\mu$m TiO$_2$ particles with a silica core (silica-TiO$_2$).

### *Results and discussion*

To allow the particles to align at the air-water interface prior to UV irradiation, they were first functionalised with FAS or HDTMS. These silanes were inherently superhydrophobic, owing to the fluorocarbon chains on FAS, and the long-chain alkylsilanes on HDTMS (E. Hoque, et al., J. Phys. Chem. C 2007, 111, 3956-3962). Theoretical calculations of the flotation behaviour of individual FAS-TiO$_2$ particles were made based on the model by Kowalczuk *et* al. (P. B. Kowalczuk, et al., Colloids Surf. Physicochem. Eng. Aspects 2012, 393, 81-85). For FAS-TiO$_2$, a range of floating positions at the interface was examined, revealing positive overall forces that indicated the dominance of the upward (floating) forces over the downward force. This was also observed experimentally: FAS- and HDTMS-functionalised particles had remained at the air-water interface even after a period of stirring.

The proposed mechanism for the degradation of FAS during UV irradiation is summarised in Fig. 1b. When the particles were irradiated by UV, hydroxyl radicals generated by the photocatalytic semiconductor particles and atmospheric water attacked the weakest part of the chain, which was at the bridging -CH$_2$ groups situated near the head of the fluoroalkyl chain. This oxidation process resulted in cleavage of the rest of the chain, leaving the particle surface akin to an SiO$_2$ surface terminated with hydroxyl groups, and thus hydrophilic. The same mechanism can be applied to HDTMS. However, instead of oxidation at the head group, the hydroxyl radicals attack along the alkyl chain in a top-down fashion, shortening them gradually. With the particles assembled at the air-water interface and the UV lamp directly above them, Janus-like modifications were made to the particles by photocatalytic decomposition of hydrophobic silanes only on the surfaces oriented towards the light. Consequently, only the FAS or HDTMS coating on such surfaces will be photodegraded.

### Example 2. Preparation of Janus particles *via* UV irradiation of the particles assembled in a Pickering emulsion using paraffin wax

The Janus particles of the current invention can also be prepared *via* UV irradiation of the particles assembled in Pickering emulsion in the presence of paraffin wax. The three main steps are as shown in Fig. 2.

In the first step, FAS-TiO$_2$ particles **12** (prepared in accordance to the general method described above) were dispersed on the surface of water **14** and heated to 75°C. This was then followed by the addition of paraffin wax flakes **22** (1:10 wax-water ratio by weight) into the mixture. The system was equilibrated at 75°C for the wax to melt completely, then subjected to vigorous mixing using the T 18 digital Ultra-Turrax disperser (IKA) at 15,000 rpm for 2 mins. The resulting mixture was immediately left to cool, yielding wax droplets **23** that were recovered *via* filtration.

In the second step, the wax droplets **23** were dispersed in water **14,** and then placed under a UV lamp **16** (wavelength 365 nm, irradiance of approximately 72 mW/cm$^2$) for 1 and 2-hr durations, while the water phase was kept under constant stirring at 100 rpm. The surface exposed to the UV irradiation underwent photocatalytic decomposition of the hydrophobic silanes, resulting in the formation of hydrophilic surfaces **18.** At the end of UV irradiation and in the final third step, the modified wax droplets **25** were filtered out and dispersed in xylene **24** to dissolve the wax core **22.** The obtained asymmetric Janus particles **27** were recovered *via* centrifugation, then washed twice in methanol *via* centrifugation and redispersion, before being left to dry in ambient conditions overnight.

The same procedure was applicable and has been demonstrated on triethoxy(octyl)silane (OTES) modified TiO$_2$ particles, FAS-treated 1 $\mu$m sicastar$^{™}$ spheres (micromod Partikeltechnologie GmbH) with silica-TiO$_2$ core-shell structures.

The three types of wax droplets formed using the respective emulsifiers (FAS-TiO$_2$, OTES-TiO$_2$, FAS-silica TiO$_2$) were filtered and dispersed over the surface of water, then placed under UV, which degraded the coating over the exposed particle surfaces. On the contrary, the surfaces buried in wax were not exposed to UV, and thus coatings on such surfaces were intact. The obtained asymmetric Janus particles, which consisted of particles containing partially coated FAS or OTES, were recovered by dissolving the wax in xylene.

The formed wax droplets **25** were observed under FESEM operating at 5 keV under secondary electron imaging mode (JEOL JSM-5500LV, JEOL JSM-6340F). Prior to visualisation, the wax droplets were dispersed on a conducting carbon tape, then sputtered with an approximately 10 nm layer of gold. The mean size of the wax droplets was counted by averaging the diameter of 20 different droplets.

**Example 3. Preparation of Janus particles *via* UV irradiation of the particles assembled in a water-in-oil Pickering emulsion**

The Janus particles of the current invention can also be prepared *via* UV irradiation of the functionalised particles assembled in a water-in-oil Pickering emulsion. The two main steps are as shown in Fig. 3.

In the first step, water-in-oil (w/o) Pickering emulsions were made, using silicone oil **26** and water **14** in an oil:water volume ratio of mixture 90:10, and 1% weight of the FAS-functionalised particles **12** (relative to the entire system). The functionalised particles were first prepared in accordance to the general method described above. Typically, FAS-$TiO_2$ was first dispersed in silicone oil **26** through sonication, then while under vigorous mixing using the T 18 digital Ultra-Turrax disperser (IKA) at 15,000 rpm, water **14** was added dropwise. The mixing proceeded for another 2 mins after all the water was added to complete emulsification. The resulting emulsions were viewed and captured under dark field optical microscopy (Olympus BX51, Infinity Analyze), and the droplet diameters were analysed and determined through ImageJ. Other volume ratios were also made, namely 10:90, 25:75, 50:50, and 75:25 (oil:water).

In the second step, the w/o emulsions were dispersed over a dish containing water **14,** and then placed under a UV lamp **16** (wavelength 365 nm, irradiance of approximately 72 mW/cm$^2$) for 30 minutes, while the water phase was kept under constant stirring at 100 rpm. The surface exposed to the UV irradiation underwent photocatalytic decomposition of the hydrophobic silanes, resulting in the formation of hydrophilic surfaces **18**. After irradiation, the Janus particles **28** were recovered through centrifugation, then washed in xylene and methanol and left to dry in ambient conditions overnight.

Due to the high amphiphobicity of FAS, FAS-$TiO_2$ particles naturally take the interface position between oil and water, and thus can act as Pickering emulsifiers. In an immiscible silicone oil-water system containing these particles, when energy was input into the system (in the form of high-energy mixing *via* the Ultra-Turrax), transient droplets form within the continuous (bulk) phase, and the particles take position at the droplet edges, eventually stabilising these droplets when an adequate droplet coverage is attained.

Both oil/water (o/w) and water/oil (w/o) emulsions were possible, though w/o emulsions were more favoured, giving rise to smaller-sized (and hence more stable) emulsion droplets. This is in line with Bancroft's rule; the slight preference of FAS for silicone oil over water resulted in silicone oil forming the continuous phase, while water formed the dispersed phase encapsulated in droplets. Phase inversion into o/w emulsions was also possible through limiting the amount of oil, leading to larger-sized emulsion droplets. All emulsions (w/o and o/w) also remained stable for at least a month, which also suggests the potential applications of FAS-$TiO_2$ as emulsifiers beyond just employing them to create Janus particles.

The resulting Pickering w/o emulsions stabilised by FAS-$TiO_2$ were observed under dark-field optical microscopy (Fig. 11a). The emulsion droplets were spherical and had an average diameter of 3.44 $\mu$m, which was in the expected size range produced by the Ultra-Turrax. Fig. 11b shows the effect of varying the volume fraction of silicone oil: 10%, 25%, 50%, 75% and 90% oil were examined. The emulsion droplets decreased in size upon increasing the volume fraction of oil, indicating higher emulsion stability. Furthermore, at 10% and 25% oil, mainly oil-in-water (o/w) emulsions were formed, i.e., water formed the continuous phase while silicone oil formed the dispersed phase. This was due to the limited availability of oil, which triggered phase inversion from w/o to o/w emulsions (Binks, B. et al., Langmuir 2000, 16, 2539-2547). These o/w emulsions creamed due to the lower density of oil, though they remained stable for at least a month (Fig. 11b, inset). Similarly, the w/o emulsions (formed at 50%, 75%, 90% oil) were also stable for at least a month, although sedimentation was observed, which was expected due to the greater density of water.

Irradiation of the emulsion droplets was carried out over the surface of water, which served to spread the emulsion droplets to allow for more efficient UV exposure. When irradiated by UV, the FAS coating over the exposed particles surfaces (those oriented outwards towards oil) were photodegraded, while the FAS coating on the unexposed surfaces (those oriented inwards towards water) intact. As a result, Janus-like particles consisting of $TiO_2$ particles with FAS on one area were obtained (FAS-$TiO_2$-UV).

**Example 4. Wettability of the as-prepared Janus particles characterised by static contact angle measurements of water on powder film made from the particles**

For practical purposes, the bulk behaviour of the as-prepared Janus particles of the current invention was investigated. The overall wettability of the Janus particles was examined through static contact angles of water droplets atop powder films made out of the particles. Contact angles of the different substrate-silane combinations before and after irradiation were measured in accordance to the method described above.

Janus particles of Example 1

*Initial studies*

It was observed that surface functionalisation of the $TiO_2$ particles with FAS rendered the particles amphiphobic, and their powder films exhibited high static contact angles of 144° against water (Fig. 4b). Powder films made from untreated $TiO_2$ particles, on the other hand, exhibited low contact angles of 20° (Fig. 4a). The Janus particles, when irradiated for varying durations, exhibited different changes in static contact angles against water (Fig. 4c and d). It was observed that the average contact angles fell to 104° after 1 hr of irradiation, and 31° after 2 hrs of irradiation, which was close to untreated $TiO_2$ values. It was postulated that partial photodegradation of the FAS had occurred, which caused the contact angles to fall, though not to the extent of untreated $TiO_2$ values. With an increase in the irradiation duration, it was likely that particle movement throughout the irradiation process had exposed more areas of the particles to the UV, which led to more areas of the particles subjected to photodegradation. As such, this caused the contact angle for the sample with 2-hr irradiation to be much lower than that in the 1-hr. Given this, it is apparent that particle wettability may be easily engineered through the control of UV intensity and duration.

As a control, the same procedure was carried out on a glass substrate (Fig. 5). Glass slides were coated with FAS, followed by irradiation under UV. In addition, a different alkylsilane, HDTMS, was used to modify the anatase $TiO_2$ particles (Fig. 6). Different substrates were used: 1 $\mu$m sized particles consisting of a silica core and an outer shell of amorphous $TiO_2$ (silica-$TiO_2$, Fig. 7a and b), ZnO (Fig. 7c and d), and octahedral $Cu_2O$ (Fig. 7e and f) were coated with FAS and subjected to irradiation as well. The fully-coated HDTMS and FAS particles were also subjected to UV irradiation.

Table 1 summarises the irradiation durations and the contact angle measurements of all experiments. Aside from the glass control, silica-$TiO_2$, and $Cu_2O$ experiments, all other samples showed a significant decrease in contact angle within the specified UV irradiation period in comparison to samples prepared using the non-irradiated particles. As the significant changes were only observed in anatase $TiO_2$ and ZnO (in which both are photocatalysts), this indicates that the presence of a photocatalyst is necessary for the accelerated photodegradation of the FAS. However, it was also observed that there was a decrease in contact angles for the glass control and silica-$TiO_2$ particles after irradiation, probably due to the photodegradation of treated organic layer by UV, regardless of the presence of photocatalysts. The mechanism for such a photodegradation had been established (Ye, T. et al., J. Phys. Chem. B 2005, 109, 9927-9938). In the case of $Cu_2O$, while there was no significant change in wettability, however, the gold NP attachment results (see Example 5) affirmed that the FAS layer had been degraded on its surface.

**Table 1.** Initial static contact angle measurements of water against various substrates before and after UV irradiation.

| Substrate | Coating | Contact angle before UV (°) | Duration of UV (h) | Contact angle after UV (°) |
|---|---|---|---|---|
| Glass slides (control) | | 119 $\pm$ 1 | 2 | 111 $\pm$ 0 |
| Anatase $TiO_2$ | FAS | 144 $\pm$ 1 | 1 | 104 $\pm$ 1 |
| | | | 2 | 31 $\pm$ 3 |
| | HDTMS | 149 $\pm$ 1 | 1 | 35 $\pm$ 3 |
| Silica-$TiO_2$ | | 138 $\pm$ 1 | 2 | 129 $\pm$ 4 |
| ZnO | FAS | 142 $\pm$ 2 | 3 | 53 $\pm$ 4 |
| $Cu_2O$ | | 151 $\pm$ 2 | 2 | 149 $\pm$ 1 |

*Subsequent studies*

The subsequent measurements of the static contact angles of the different substrate-silane combinations before and after irradiation are summarised in Table 2 and as shown in Fig. 8 After irradiation for 1 hr, both FAS-$TiO_2$-UV1h and HDTMS-$TiO_2$-UV1h exhibited lower contact angles than FAS-$TiO_2$ and HDTMS-$TiO_2$ (respectively), with HDTMS-$TiO_2$-UV1h exhibiting a contact angle (34.9°) closer to untreated $TiO_2$. Prolonged irradiation of FAS-$TiO_2$ for another hour (FAS-$TiO_2$-UV2h) was required to attain a similar value (30.5°). This implied a greater UV stability of the fluoroalkyl chains on the particle surface relative to the alkyl chains.

The compacted $Cu_2O$ particles did not exhibit significant changes in contact angle after irradiation. This might be due to the octahedral shape of the particles, which maintain a higher degree of microscopic roughness in the powder film. Such a rough surface could maintain a high contact angle because of the trapped air on the surface structure, creating a heterogeneous surface following the Cassie-Baxter mechanism. Nevertheless, photocatalytic degradation of FAS on $Cu_2O$ had occurred and was verified by the gold labelling (see Example 5). Similarly, due to the microscopic roughness of the powder film, untreated $Cu_2O$ had an apparent contact angle of 67.1°, which was likely lowered by the roughness factor following the Wenzel equation.

For comparison, glass substrates were also treated with FAS and subjected to UV irradiation for 2 hrs. FAS treatment rendered the glass substrates superhydrophobic with a high contact angle of 119.1°. For smooth surfaces such as the glass substrates used here, 120° is the maximum contact angle attainable. Contrary to the FAS-$TiO_2$ particles, even after irradiation of the glass substrates for 2 hrs, only a slight decrease in the contact angle was observed, which implies that most of the fluoroalkyl chains on the glass substrates were intact. This affirms that the presence of a photocatalyst accelerates the degradation of FAS to bring about larger changes in wettability. Nevertheless, degradation is still feasible for non-photocatalytic surfaces when subjected to a higher UV irradiance and/or longer irradiation durations.

**Table 2.** Subsequent static contact angle measurements of water against various substrates before and after UV irradiation.

| Substrate | Contact angle (°) |
| --- | --- |
| Untreated $TiO_2$ | 19.9 ± 2.3 |
| FAS-$TiO_2$ | 146.9 ± 1.0 |
| FAS-$TiO_2$-UV1h | 104.6 ± 2.0 |
| FAS-$TiO_2$-UV2h | 30.5 ± 3.0 |
| HDTMS-$TiO_2$ | 148.9 ± 1.0 |
| HDTMS-$TiO_2$-UV1h | 34.9 ± 2.6 |
| Untreated $Cu_2O$ | 67.1 ± 0.1 |
| FAS-$Cu_2O$ | 151.3 ± 2.2 |
| FAS-$Cu_2O$-UV2h | 148.9 ± 1.4 |
| Untreated glass | 59.6 ± 0.8 |
| FAS-glass | 119.1 ± 1.1 |
| FAS-glass-UV2h | 111.3 ± 0.4 |

Janus particles of Example 2

The Pickering emulsification of wax droplets using FAS-$TiO_2$ particles was achieved with droplet sizes having a mean diameter of 28 $\mu$m (Fig. 9a). The FAS coating over the particles rendered them superhydrophobic with contact angles of 149° against water, and oleophobic with contact angles of 142° against mineral oil (Fig. 10). While emulsifiers typically contain partial wettability of both oil and water phases, these amphiphobic particles, which do not partition into either phases, have a tendency to assemble at the oil-water interface. As such, when a sufficiently high energy was input in the heated, molten wax-water system, the particles naturally took position at the interfaces of any droplets formed. In this case, since wax hardens at room temperature, cooling of the droplets allowed the particles to anchor at the interfaces. The resulting droplets were markedly spherical and consisted of a wax core with FAS-$TiO_2$ particles assembled over the surface, partially buried in wax.

The OTES-$TiO_2$ particles were also used for the formation of wax droplets (Fig. 9b and c). The contact angles exhibited by OTES-$TiO_2$ powder films were 141° and 28° against water and mineral oil respectively (Fig. 10), showing favourable wettability towards oil as compared to water. Typically, emulsifiers contain partial wettability of both oil and water, and the shorter alkyl chain of the OTES coating is considered to be more favourable towards water and oil as opposed to the FAS coating. The wax droplets formed by OTES-$TiO_2$ particles had a mean diameter of 34 $\mu$m, which did not differ much from those formed using FAS-$TiO_2$.

In a similar procedure, FAS-silica $TiO_2$ particles were also used as Pickering emulsifiers for the wax droplets. These particles gave contact angles of 145° against water and 45° against mineral oil (Fig. 10). The resulting wax droplets were also spherical in appearance and had a mean diameter of 73 $\mu$m (Fig. 9d). This was significantly larger than that produced using FAS-$TiO_2$ particles due to the larger size of the particles (1 $\mu$m in diameter).

After UV irradiation, the water contact angle of the recovered asymmetric Janus particles decreased. The FAS-$TiO_2$ particles had a mean static contact angle of 120° after 1 hr of UV irradiation, and an angle of 53° after 2 hrs of irradiation. The mean static contact angles of OTES-$TiO_2$ particles after irradiation for 1 hr and 2 hrs were 136° and 69°, respectively, and 127° and 112° respectively, for FAS-silica $TiO_2$ (Fig. 10). Table 3 summarises the static contact angles for FAS-$TiO_2$, OTES-$TiO_2$ and FAS-silica $TiO_2$ before and after different irradiation durations.

**Table 3.** Subsequent static contact angle measurements of water against various substrates before and after UV irradiation.

| Substrate | Contact angle before UV (°) | Duration of UV (h) | Contact angle after UV (°) |
|---|---|---|---|
| FAS-TiO$_2$ | $149 \pm 2$ | 1 | $120 \pm 8$ |
| | | 2 | $53 \pm 4$ |
| OTES-TiO$_2$ | $141 \pm 2$ | 1 | 136 |
| | | 2 | $69 \pm 4$ |
| FAS-silica TiO$_2$ | 145 | 1 | $127 \pm 1$ |
| | | 2 | $112 \pm 3$ |

Janus particles of Example 3

For FAS-TiO$_2$, the relatively lower contact angle of silicone oil (135.6°) compared to water (145.1°) indicated a slight preference of FAS-TiO$_2$ for silicone oil over water. Consequently, water-in-oil emulsions were formed, following the Bancroft rule, which states that the phase in which the emulsifier is more soluble constitutes the continuous phase. In addition, the use of a large volume fraction of silicone oil (9:1 oil:water) also further promoted the formation of w/o emulsions. After 30 mins of UV irradiation, the water contact angle of FAS-TiO$_2$-UV had decreased from 145.1° to 126.7° (Table 4), indicating less amounts of FAS on the surface of these particles. Silicone oil contact angles had decreased by a greater margin from 135.6° to 39.7°, though this was likely contributed by traces of silicone oil left on the particles. The static contact angles of silicone oil and water against various particle types are summarised in Table 4.

**Table 4.** Static contact angles of water and silicone oil on powder films of various types of TiO$_2$ particles.

| Substrate | Liquid | Contact angle (°) |
|---|---|---|
| Untreated TiO$_2$ | Water | $19.9 \pm 2.3$ |
| | Silicone oil | $16.7 \pm 0.5$ |
| FAS-TiO$_2$ | Water | $145.1 \pm 1.1$ |
| | Silicone oil | $135.6 \pm 2.5$ |
| FAS-TiO$_2$-UV | Water | $126.7 \pm 6.3$ |
| | Silicone oil | $39.7 \pm 5.8$ |

**Example 4. Characterisation of the as-prepared Janus particles by Fourier-transform infrared spectroscopy (FTIR) and thermogravimetric analysis (TGA)**

*Fourier-transform infrared spectroscopy (FTIR)*

The Janus particles prepared from Example 1 were analysed by FTIR spectroscopy and their spectra are as shown in reported in Fig. 12. All TiO$_2$ particles (untreated, as well as FAS- and HDTMS-functionalised before and after UV) showed a strong peak at 700 cm$^{-1}$ that corresponded to the stretching vibration of Ti-O. Broad bands at 3435 cm$^{-1}$ corresponding to the stretching vibration of -OH, and sharper bands at 1630 cm$^{-1}$ corresponding to surface adsorbed water were also present in all of them (E. Ukaji, et al., Appl. Surf. Sci. 2007, 254, 563-569; S. Pazokifard, et al., Adv. Powder Technol. 2012, 23, 428-436). Similarly, all Cu$_2$O particles (untreated, FAS-Cu$_2$O, and FAS-Cu$_2$O-UV2h) showed similar broad bands at 3435 cm$^{-1}$ and sharper bands at 1630 cm$^{-1}$. The strong peak observed in all Cu$_2$O particles at 630 cm$^{-1}$ corresponded to the Cu-O deformation vibration (D.-F. Zhang, et al., J. Mater. Chem. 2009, 19, 5220-5225).
Relative to the particle bands, FAS and HDTMS peaks were expectedly weaker in intensity. FAS shows peaks in the 1100-1300 cm$^{-1}$ region pertaining to C-F$_2$ and C-F$_3$ bonds (J.-D. Brassard, et al., Appl. Sci. 2012, 2, 453-464). The peaks at 1205 cm$^{-1}$ and 1145 cm$^{-1}$ (magnified in the insets of Fig. 12a and c) corresponding to the C-F$_2$, C-F$_3$, and Si-O-C bonds were present in both FAS-TiO$_2$ and FAS-Cu$_2$O, and not in their untreated counterparts, affirming the uptake of FAS on these particles. These peaks were also weaker in their irradiated counterparts (FAS-TiO$_2$-UV1 h, FAS-Cu$_2$O-UV2h), affirming the degradation of FAS. HDTMS peaks appear in the 2800-3000 cm$^{-1}$ region, which are due to the stretching vibration of C-H from C-H$_2$ and C-H$_3$ in the alkyl chain (X. Wang, et al., Holzforschung 2013, 67, 667-672). Two peaks present at 2850 cm$^{-1}$ and 2920 cm$^{-1}$ were seen for both HDTMS-TiO$_2$ and HDTMS-TiO$_2$-UV1h (Inset in Fig. 12b). These peaks were also weaker in the latter, which was consistent with less HDTMS due to degradation.

*Thermogravimetric analysis (TGA)*

TGA was performed on the as-prepared Janus particles of the current invention (of Examples 1-3) to provide a quantitative comparison of the surface coating (FAS and HDTMS) coating on the particles. At high temperatures, fluoroalkylsilanes thermally decompose at the methylene chain (T. Monde, et al., J. Ceram. Soc. Jpn. 1996, 104, 682-684), and similarly for alkylsilanes, this occurs at the CAC bond adjacent to the Si atom (G. J. Kluth, et al., Langmuir 1997, 13, 3775-3780). The amount of coating over the particles was inferred from weight losses occurring during the thermal decomposition in a nitrogen atmosphere.

Janus particles of Example 1

• *Initial analysis*

In the initial studies, TGA was performed on the $TiO_2$ particles before and after irradiation, and their respective weight losses are summarised in Table 5. The particles were subjected to a temperature ramp up to 800°C in a nitrogen atmosphere. The respective weight loss curves with respect to temperature are shown throughout Fig. 13a and b. Heating beyond 400°C results in the decomposition of the silane coating over the particle surface (Campos, R. et al., Langmuir 2011, 27, 10206-10215). As such, the weight loss is linked to the amount of coating present, and a lower weight loss would account for less amounts of coating.

In the case of the anatase $TiO_2$ substrate, an average onset temperature of 438°C and 401°C was observed for FAS and HDTMS respectively. At such temperatures, the weight losses for both FAS and HDTMS were lower after they were UV treated when compared to their non-UV counterparts. This indicated quantitatively less amounts of FAS and HDTMS coating on the particles after UV. The results all agreed that UV irradiation had brought about a degree of photodegradation of the silane coating.

**Table 5.** Weight loss results from thermogravimetric analysis of the particles before and after UV irradiation.

| Substrate | Coating | Duration of UV (h) | Average onset temperature (°C) | Weight loss before UV (%) | Weight loss after UV (%) |
|---|---|---|---|---|---|
| Anatase $TiO_2$ | FAS | 1 | 438 | 4.39 | 3.86 |
| | HDTMS | 1 | 401 | 1.63 | 1.19 |

• *Subsequent analysis*

In subsequent TGA, untreated particles (both $TiO_2$ and $Cu_2O$), as well as FAS- and HDTMS-functionalised particles before and after UV irradiation were analysed. The results are summarised in Table 6, and the corresponding weight loss curves are as shown in Fig. 14. Only one distinct slope was observed for both FAS- and HDTMS-functionalised particles, and this slope was present before and after irradiation. The onset temperature averaged around 328.5 °C for FAS-$TiO_2$ and 316.1 °C for FAS-$Cu_2O$, while HDTMS-$TiO_2$ had an average onset temperature of 399.3 °C. Untreated $TiO_2$ showed an insignificant weight loss of 0.05% at that region, affirming that the weight losses were attributed to the FAS and HDTMS coatings. Untreated $Cu_2O$ showed a 0.51% weight loss, but at an onset point of 442.1 °C, which is above the temperature region where weight loss had occurred for FAS. In addition, there were similar weight losses in that region for both FAS-$Cu_2O$ and FAS-$Cu_2O$-UV2h. This was likely due to the oxidation of $Cu_2O$ into CuO.

In all three cases, the onset temperature for FAS and HDTMS decomposition of the UV-irradiated particles were lower than their fully-functionalised counterparts. Lower weight losses were also observed for particles that were irradiated for all cases (FAS-$TiO_2$-UV1h, HDTMS-$TiO_2$-UV1h, and FAS-$Cu_2O$-UV2h), which indicate less amounts of coating on particles that had been irradiated. This verifies that the irradiation process had partially degraded the coating on the particles. HDTMS-$TiO_2$-UV1h also showed a proportionally larger weight loss than FAS-$TiO_2$-UV1h, which was consistent with the relatively larger decrease in contact angle after 1 hr of irradiation.

**Table 6.** Onset temperature and weight loss data generated from TGA of various particles.

| Substrate | Onset temperature (°C) | Weight loss (%) | Loss attributed to |
|---|---|---|---|
| Untreated $TiO_2$ | 369.0 | 0.05 | - |
| FAS-$TiO_2$ | 345.9 | 4.39 | FAS |

(continued)

| Substrate | Onset temperature (°C) | Weight loss (%) | Loss attributed to |
|---|---|---|---|
| FAS-TiO$_2$-UV1h | 311.1 | 3.86 | FAS |
| HDTMS-TiO$_2$ | 411.6 | 1.63 | HDTMS |
| HDTMS-TiO$_2$-UV1h | 386.8 | 1.19 | HDTMS |
| Untreated Cu$_2$O | 442.1 | 0.51 | Oxidation into CuO |
| FAS-Cu$_2$O | 324.1<br>448.2 | 7.21<br>0.21 | FAS<br>Oxidation into CuO |
| FAS-Cu$_2$O-UV2h | 308.0<br>442.5 | 5.45<br>0.56 | FAS<br>Oxidation into CuO |

Janus particles of Example 2

Thermogravimetric analysis (TGA) performed on FAS-TiO$_2$ and OTES-TiO$_2$ particles gave quantitative insight into the amount of FAS and OTES coating present on the particle surfaces. Weight losses of the particles before and after 2-hr UV irradiation periods are summarised in Table 7, and their respective weight loss curves are shown in Fig. 15a and b. Average onset temperatures of 441°C and 501°C were observed for FAS and OTES respectively. Weight loss corresponding to the amount of coating present on the particle surfaces was measured across the slopes at these temperatures. In both cases, the weight loss for FAS and OTES were lower after UV irradiation, as compared to their counterparts that were not UV irradiated (i.e, full coating intact), therefore affirming quantitatively the reduced amounts of FAS and OTES coating on the particles after UV irradiation.

**Table 7.** Weight loss results from thermogravimetric analysis of the particles before and after UV irradiation.

| Substrates | Duration of UV (hr) | Average onset temperature (°C) | Weight loss before UV (%) | Weight loss after UV (%) |
|---|---|---|---|---|
| FAS-TiO$_2$ | 2 | 441 | 2.52 | 1.30 |
| OTES-TiO$_2$ | 2 | 501 | 1.97 | 1.55 |

Janus particles of Example 3

Thermogravimetric analysis (TGA) was performed to assess quantitatively the amount of FAS on the particle surfaces. The weight losses occurring during the temperature ramp corresponded to the decomposition of materials throughout the process. Table 8 details the weight loss and their onset temperatures of the TGA performed, and Fig. 16 shows the weight loss curves for the three sets of particles examined. Untreated TiO$_2$ showed no distinct weight loss, while FAS-TiO$_2$ and FAS-TiO$_2$-UV showed one distinct weight loss slope each, verifying that the weight loss was due to the decomposition of FAS. The onset temperature for FAS decomposition on FAS-TiO$_2$ was 366.86°, while the onset temperature for FAS-TiO$_2$-UV was higher at 414.49°, likely due to traces of silicone oil on the particles. However, the weight loss for FAS-TiO$_2$-UV was lower at 0.92%, relative to 1.44% on FAS-TiO$_2$, which was representative of the weight loss of a fully-coated FAS-TiO$_2$. Following UV irradiation, the lower weight loss affirmed quantitatively less amounts of FAS on the particles.

**Table 8.** Weight loss results from thermogravimetric analysis of the particles before and after UV irradiation.

| Substrates | Average onset temperature (°C) | Weight loss (%) |
|---|---|---|
| FAS-TiO$_2$ | 366.86 | 1.44 |
| FAS-TiO$_2$-UV | 414.39 | 0.92 |

**Example 5. Functionalisation of the as-prepared Janus particles with gold nanoparticles and characterisation by field-emission scanning electron microscopy (FESEM) and energy dispersive spectroscopy (EDS)**

In order to visualise the distribution of coating over the particle surface after UV irradiation, the particles irradiated by UV were further modified with APTES and subsequently with gold nanoparticles (gold NPs). The schematic for this procedure is as shown in Fig. 17. Since the UV-irradiated surfaces **18** were silica-like surfaces, they could be subjected to further modification with other alkylsilanes to form 30. In contrast, the FAS surfaces **12** that were still intact after irradiation, therefore surface **12** are not able to be further modified with other silanes. The affinity of the terminal amine groups on APTES for gold NPs has been well established (K. C. Grabar, et al., Anal. Chem. 1995, 67, 735-743). Therefore, the attachment of gold NPs **32** on the surface would facilitate the visualisation of APTES-functionalised surfaces **30.** With that, irradiated particles were functionalised with APTES and gold NPs were used to visualise irradiated surfaces on the particles.

*Method (gold nanoparticle labelling)*

Typically, $TiO_2$, FAS-$TiO_2$, FAS-$TiO_2$-UV1h, $Cu_2O$, FAS-$Cu_2O$, and FAS-$Cu_2O$-UV2h particles were each treated with APTES following a similar surface functionalisation procedure. Untreated particles ($TiO_2$, $Cu_2O$) fully functionalised with APTES were used as the positive control sample, while FAS-functionalised particles not subjected to UV (FAS-$TiO_2$, FAS-$Cu_2O$) and functionalised with APTES were used as the negative control sample. A dispersion of particles (1 mg/mL) in ethanol was first prepared. 1% v/v APTES in ethanol was added dropwise into the dispersion, and the resulting solution was stirred for 1 hr and heated at 100 °C for 1 hr. The particles were recovered, washed twice in ethanol to remove excess APTES, and then dispersed in water. The gold nanoparticle (NP) solution (10 nm gold NP for $TiO_2$ particles, 30 nm gold NP for $Cu_2O$ particles) was directly added to the aqueous particle solution in a pre-determined volume ratio. For example, 10 nm gold NP suspended in citrate buffer in a 1:4 (particle:gold NP) volume ratio was used. The system was sonicated at 37 kHz (Elmasonic S30H, Elma) for 1 hr and left to stand overnight. The recovered particles were then washed twice in water to remove un-adsorbed gold NPs. The particles were drop cast on aluminum foil, and then observed under FESEM (JEOL JSM-7600F) operating at an accelerating voltage of 10 keV in secondary electron imaging mode. As controls, untreated particles and particles fully coated with FAS were also treated with APTES and submerged in gold NP solution in the same procedure.
Other particles prepared from Examples 1-3 were treated using the same procedure described herein.

*Results and discussion*

Janus particles of Example 1

Under FESEM (Fig. 18), gold NPs were distributed over entire surfaces of the untreated (full APTES) $TiO_2$ particles. On the contrary, almost no gold NPs were found on the FAS-$TiO_2$ particles, verifying that the fluoroalkyl chains have no affinity for gold NPs. On the irradiated particles (FAS-$TiO_2$-UV1h), gold NPs attached preferentially to certain areas, revealing the Janus boundaries of each particle. Various Janus ratios were also seen; some with more gold NP adhesion (less FAS) and some with less gold NP adhesion (more FAS).
To affirm that the tiny particles on the surface of the larger particles were gold NPs, EDS was performed on FAS-$TiO_2$-UV1h (Fig. 18d, Table 9). Relative to the two controls, this also affirmed the uptake of APTES by irradiated particles - degradation of the FAS previously on those areas must have occurred in order to allow the uptake of APTES. Particle loadings for $TiO_2$ and gold NPs were kept proportionally consistent throughout the three experiments, and excess unbound gold NPs were also seen on the aluminum foil substrate in the case of FAS-$TiO_2$-UV1h (Fig. 18d). This has verified that the unexposed regions on the particles with remaining FAS have no affinity with gold NPs, while the regions that have been exposed to UV see the gold NP attachment. This has allowed an approximate visualisation of the Janus boundaries on the individual $TiO_2$ particles.

**Table 9.** EDS elemental analysis of FAS-$TiO_2$-UV1h. Aluminum foil was used as the substrate for drop cast of the particle solution.

| Element | Weight % | Atomic % |
| --- | --- | --- |
| C K | 4.64 | 9.94 |
| O K | 2.73 | 4.39 |
| Al K | 87.54 | 83.53 |
| Si K | 0.92 | 0.84 |
| Ti K | 0.46 | 0.25 |
| Fe K | 1.71 | 0.79 |
| Au M | 2.01 | 0.26 |

(continued)

| Element | Weight % | Atomic % |
| --- | --- | --- |
| Total | 100.00% | 100.00% |

A similar gold NP attachment behaviour was observed in the case of $Cu_2O$ particles (Fig. 19). Similarly, FAS-$Cu_2O$ had no affinity for gold NPs, untreated $Cu_2O$ (fully APTES coated) showed extensive gold NP attachment, while the irradiated (FAS-$Cu_2O$-UV2h) particles showed gold NPs preferentially attached at certain areas of the octahedral shaped particles. In contrast to the untreated $Cu_2O$ particles, FAS-$Cu_2O$-UV2h displayed a distinct Janus appearance. Although there was no observed change in wettability after irradiation, the attachment of gold NP on the irradiated $Cu_2O$ affirmed that photodegradation had occurred. It is likely that extended irradiation durations were required for a more significant change in wettability for these particles. Octahedral $Cu_2O$ had been reported to demonstrate photocatalytic ability (Chu, C.-Y.; Huang, M. H., J. Mat. Chem. A 2017, 5, 15116-15123). Evidently, the relatively lower degradation in comparison to $TiO_2$ and ZnO is due to its weaker photocatalytic ability. Two possibilities are presented: Chu *et al.* found that the octahedral form of $Cu_2O$ possessed fewer electron holes at the surface, thereby reducing the occurrence of oxidation reactions to generate hydroxyl radicals, which are vital for the photodegradation of the FAS. Another possibility is that the particular mechanisms in generating the radicals for $Cu_2O$ is different from $TiO_2$ and ZnO.

The silica-$TiO_2$ particles were also subjected to gold NP labelling using 30 nm gold nanoparticles (Fig. 20a and b). Similarly, Janus boundaries could be seen for these particles, with the gold NP preferentially attaching to certain areas. Despite the small decrease in contact angle after irradiation, the preferential gold nanoparticle distribution on their surfaces verified that partial photodegradation had occurred.

In the case of ZnO particles (Fig. 20c), gold NP distribution was significantly extensive and appears consistent with the relatively low contact angle of 53° as reported earlier. This was probably due to the extended irradiation period of 3 hrs, in which more ZnO particle surfaces were irradiated, therefore reducing the overall amounts of FAS which then allowed more sites for gold NP attachment.

Janus particles of Example 2

The gold NPs labelling procedure was also carried out on the Janus particles of Example 2. To demonstrate the differences, two controls were used: FAS-$TiO_2$ particles that had not been irradiated by UV were used as the negative control, while $TiO_2$ particles fully treated with APTES (APTES-$TiO_2$) were used as the positive control.

Both controls, as well as the FAS-$TiO_2$ and OTES-$TiO_2$ particles that have been irradiated for 1 hr were treated with APTES and gold NPs, and subsequently examined under SEM (Fig. 21). It was observed in Fig. 21b that the surfaces of the APTES-$TiO_2$ particles (positive control) were extensively dotted with the smaller 10 nm gold NP. Conversely, no gold NP was found on the FAS-$TiO_2$ particles (negative control), implying no affinity of the FAS coating with gold nanoparticles (Fig. 21a). Both the irradiated FAS-$TiO_2$ and OTES-$TiO_2$ particles (Fig. 21c and d, respectively) lay in between these two extremes, with only some surfaces showing gold NPs attachment. The gold NPs distribution tended towards one hemisphere of each of these particles, showing a Janus distribution.

It was also observed that FAS-silica $TiO_2$ particles had little or no gold NP on their surfaces (Fig. 22a), while the fully APTES treated particles (Fig. 22c) had extensive gold NPs distribution over their surfaces. For the FAS-silica $TiO_2$ particles that had been irradiated by UV for 1 hr, selective gold NPs distribution was observed (Fig. 22b). In this case, the Janus boundary can also be observed more clearly as the particles were spherical.

Janus particles of Example 3

Fig. 23 shows three sets of particles (untreated $TiO_2$, FAS-$TiO_2$, and FAS-$TiO_2$-UV), following APTES functionalisation and gold NPs attachment. Untreated $TiO_2$ particles (Fig. 23c) were covered extensively by the smaller 10 nm sized gold NPs over their surfaces, affirming the uptake of APTES. Conversely, no gold NP attachment was observed on FAS-$TiO_2$ (Fig. 23a). This shows that FAS has no affinity for gold NPs, and that since FAS-$TiO_2$ particles were entirely functionalised by FAS over its entire surface, they would not be able to be functionalised with APTES and thus no gold NPs attachment would occur. Fig. 23b shows gold NPs attachment on the FAS-$TiO_2$-UV particles, but the distribution of gold NPs was sparse and had not covered over the entire particle surfaces (as in the untreated $TiO_2$ case). The gold NPs also appeared to show preferential attachment towards one side of the particles, showing a Janus-like appearance. This affirms the partial degradation of FAS on FAS-$TiO_2$-UV, which allowed functionalisation with APTES and subsequently with gold NPs.

**Example 6. Self-assembly of the as-prepared Janus particles at the oil/water interface**

To understand the behaviour of the as-synthesised Janus particles in water and oil, the oil/water partitioning of the irradiated particles was compared against non-functionalised and fully functionalised particles.

*Method (Toluene/water partitioning)*

In a glass container containing 1:1 volume ratio of toluene and water, approximately 2 mg of the particles was dispersed at the interface. The container was briefly sonicated at 37 kHz (Elmasonic S30H, Elma) for 10 s, manually shaken, and then left to stand for 15 min before photographs were taken.

*Results and discussion*

Untreated $TiO_2$, HDTMS-$TiO_2$, and HDTMS-$TiO_2$-UV1h particles were spread at the interface of an immiscible toluene/water mixture. Following disruption by sonication, untreated $TiO_2$ dispersed into the bottom water phase, while HDTMS-$TiO_2$ dispersed into the top toluene phase, verifying their respective hydrophilic and oleophilic characters (Fig. 24a). In contrast, the HDTMS-$TiO_2$-UV1h Janus particles returned to and remained at the interface, affirming their strong affinity for the toluene/water interface (Fig. 24b), because of their unique hydrophilic and hydrophobic-oleophilic character.

Fig. 25a and b show the assembly of the FAS-treated anatase $TiO_2$ particles in a toluene/water mixture, in which both the FAS-treated particles, with and without UV treatment, had assembled at the interface of the two-phase system. This was expected, owing to the amphiphobic properties of FAS. Comparatively, the UV treated particles had distributed across the interface in an even manner, while those that were not treated with UV had assembled as agglomerates at the interface. This was due to the partial photodegradation of FAS on the UV-treated particles, which allowed them to be less phobic towards both phases and hence spread out at the interface.

According to Binks, Janus particles that contain equal areas of apolar (hydrophobic) and polar (hydrophilic) regions are both surface active and amphiphilic, and the two regions are characterised by the contact angles $\theta_A$ and $\theta_P$, respectively (B. Binks, P. Fletcher, Langmuir 2001, 17, 4708-4710). The angle $\alpha$ indicates the position of the Janus boundary between the two regions, and ranges between 0° and 180° for Janus particles with varying ratios of both regions (Fig. 26), with $\alpha$ = 0° for homogeneous hydrophobic particles and 180° for homogeneous hydrophilic particles. For Janus particles with $\alpha < \theta_A$ or $\theta_P < \alpha$, they would have contact angles similar to the homogeneous counterpart of the dominant region. A 50-50 balanced Janus particle has $\alpha$ = 90°. An immersion angle $\beta$, which indicates a Janus particle's immersion depth at the oil/water interface, is a reflection of the surface free energies of a Janus particle $\beta$ (T. Ondarçuhu, et al., J. Phys. 1990, 51, 1527-1536). However, the "Janus balance" of a particle is not defined solely by the area of each region. The desorption energies of each region into oil and water must be considered, and can also vary with different oils (S. Jiang, S. Granick, Janus balance of amphiphilic colloidal particles, AIP (2007)). Assuming $\theta_A < a < \theta_P$, the Janus balance ($J$) for HDTMS-$TiO_2$-UV1h particles may be calculated using Eq. (1):

$$J = \frac{sin^2\alpha + 2cos\theta_P(cos\alpha - 1)}{sin^2\alpha + 2cos\theta_A(cos\alpha + 1)} \qquad \text{Eq. (1)}$$

$J$ is defined as the normalisation of the energy required to desorb the particle into the oil phase by that required to desorb it into the water phase. At $J$ = 1, maximum adsorption energy of the particle at the interface is attained, and thus is used to describe a perfectly balanced Janus particle. For HDTMS-$TiO_2$-UV1h, $J$ = 5.90, which indicates a dominantly hydrophilic surface that agrees with the observed bulk contact angle. For homogeneous particles, $\alpha = \theta_A = \theta_P$, and Eq. (1) may be reduced to:

$$J = \frac{(1 - cos\beta)^2}{(1 + cos\beta)^2} \qquad \text{Eq. (2)}$$

HDTMS-$TiO_2$ particles had a J value of 0.0060, while for untreated $TiO_2$ particles J = 1055.87, which agrees with the observed preference of HDTMS-$TiO_2$ particles for the toluene phase and untreated $TiO_2$ particles for the water phase. In contrast, a J value of 5.90 for HDTMS-$TiO_2$-UV1h is much closer to 1, indicating higher adsorption energy to the interface. This agrees with their attachment to the toluene/water interface seen in Fig. 24.

In the case of the FAS-treated particles, both FAS-$TiO_2$ and FAS$TiO_2$-UV1h particles remained at the interface after disruption (Fig. 25a and b). However, unlike FAS-$TiO_2$ that agglomerated at the interface, FAS-$TiO_2$-UV1h had spread out as a more even layer across the interface, due to a relatively higher affinity for both phases. For FAS-$TiO_2$-UV1h particles, $J$ = 0.77, which agrees with this observation. On the contrary, $J$ = 0.0078 for FAS-$TiO_2$ particles following Eq. (2).

While this implies that the particles have a relatively larger preference for the oil phase than FAS-TiO$_2$-UV1h, the particles remained at the interface instead. This is because Eq. (2) assumes that a hydrophobic/apolar surface is equivalent to an oleophilic one, which is not the case here. Due to the low surface energy of the FAS coating, the particles do not desorb into the oil and water phases, and is thus known to be amphiphobic. Consequently, FAS-TiO$_2$ agglomerated at the oil/water interface to minimise the total amount of particle surface area in contact with both phases. This implies that FAS particles with J values much lower than 1 are able to remain at the interface due to their repellency towards both oil and water.

Similarly, both FAS-Cu$_2$O and FAS-Cu$_2$O-UV2h particles remained at the interface. While there was no discernible difference in the way they assembled at the interface, some FAS-Cu$_2$O-UV2h particles had dispersed into the bottom water phase, which affirms their relative increase in wettability following UV irradiation (Fig. 25c). In both cases, most particles stuck on the glass walls of the container at the interface. The phenomenon of FAS-modified particles forming a climbing film on glass containers is known (Y. Lai, et al., Part. Part. Syst. Char. 2015, 32, 355-363), and is the result of the particles coming in contact with liquids that do not wet them. For homogeneous FAS-modified particles, two observations were made: FAS-TiO$_2$ particles with $J$ = 0.0078 agglomerated at the interface, while FAS-Cu$_2$O particles with $J$ = 0.0043 formed a climbing film along the walls of the container. The relatively lower $J$ value of FAS-Cu$_2$O particles indicates a larger aversion towards water than FAS-TiO$_2$, and thus elucidates their preference for adhering to the glass walls instead of agglomerating at the interface. For FAS-Cu$_2$O-UV2h particles with $J$ = 0.11, they formed a climbing film as well. Due to the microscopic roughness of these particles that contributes to an even higher desorption energy into water, the $J$ value of these particles must necessarily be closer to 1 to achieve a more "perfect" assembly at the interface (as observed in the case of FAS-TiO$_2$-UV1h particles).

In terms of stabilising emulsions, a Janus balance of 1, where desorption energies into the oil and water phase are equal, is most stable (S. Jiang, S. Granick, Janus balance of amphiphilic colloidal particles, AIP (2007)). Across the three types of Janus particles examined, FAS-TiO$_2$-UV1h was closest to 1. FAS-Cu$_2$O-UV2h has a $J$ value of only 0.11 due to the microscopic roughness of the particles, which has overstated its contact angle, leading to an exaggerated energy required to desorb the particle into water. Conversely, the HDTMS-TiO$_2$-UV1h had a $J$ value much larger than 1. In all three cases, the particles remained at the interface for at least a month when left unperturbed, showing good stability.

### Example 7. Sun protection factor of the as-prepared Janus particles at the oil/water interface

The uneven topography of the skin makes it a challenge to attain an effective protection against UV, which requires an even application of sunscreen over the skin surface. TiO$_2$ is widely used in sunscreen due to their UV-blocking properties when the particles are spread over the skin. To examine the efficacy of the Janus TiO$_2$ particles of the current invention in sunscreen, the particles were incorporated into a formulation adapted from Wiechers *et al.* (Wiechers, S. et al., Cosmetics & Toiletries 2013, 128, 2-6*)*.

For FAS-TiO$_2$ Janus particles, having a partial FAS coating confers these particles with both amphiphilic (oil and water attracting) and amphiphobic (oil and water repelling) properties. Such a configuration allows these particles to assemble favourably when incorporated in sunscreen as shown in Fig. 27, in which the amphiphobic domains of the FAS-TiO$_2$ Janus particles drives particles upwards to the surface of the formulation, while the amphiphilic domains keep the particles anchored to the formulation, also keeping the particle orientation in place. The particles form a topmost even layer over the surface when the sunscreen formulation is spread across screen, conferring various benefits.

*Method*

Table 10 lists the formulations used for the sunscreens. Different types of TiO$_2$ particles were used to make sunscreens to assess the differences between them: untreated TiO$_2$ particles (Untreated), fully coated FAS particles (FAS), FAS-Janus particles (FAS-Janus), fully coated OTES particles (OTES), OTES-Janus particles (OTES-Janus), and Blank representing no particles used. In Blank, water was added *quantum satis* to replace the weight percent of TiO$_2$ particles. The procedure was as follows: all premixes (except Premix B) were first separately made through magnetic stirring at ambient conditions. In the main vessel, Premix A was heated to 80-85°C, Premix B (TiO$_2$) was added and dispersed for 5 mins, then Premix C was added, stirring was increased, and continued for 15 mins. The mixture was left to cool with gentle stirring; once below 40°C, Premix D and Premix E was added in order. The final sunscreen was stirred for a further 15 mins to allow complete mixing.

Table 10. Formulation details (in weight %) of each sunscreen using different types of particles.

| Raw Material | Blank | Untreated | FAS | FAS-Janus | OTES | OTES-Janus |
|---|---|---|---|---|---|---|
| **Premix A** | | | | | | |
| Tego Care 215 (Evonik) | 3.5% | | | | | |

(continued)

| Raw Material | Blank | Untreated | FAS | FAS-Janus | OTES | OTES-Janus |
|---|---|---|---|---|---|---|
| **Premix A** | | | | | | |
| Stearyl alcohol | 0.5% | | | | | |
| Glyceryl stearate | 0.5% | | | | | |
| Tegosoft DEC (Evonik) | 5.0% | | | | | |
| Tegosoft liquid (Evonik) | 5.0% | | | | | |
| Tegosoft TN (Evonik) | 5.0% | | | | | |
| Xanthan gum | 0.2% | | | | | |
| **Premix B** | | | | | | |
| Treated or untreated TiO$_2$ particles | 0% | 5.0% | | | | |
| **Premix C** | | | | | | |
| Water | 74.8% | 69.8% | | | | |
| Glycerin | 3.0% | | | | | |
| **Premix D** | | | | | | |
| Carbomer | 0.2% | | | | | |
| Mineral oil | 0.8% | | | | | |
| **Premix E** | | | | | | |
| Sodium hydroxide | 0.5% | | | | | |
| Phenoxyethanol (and) Ethylhexylglycerin | 1.0% | | | | | |

To assess the Sun Protection Factor (SPF) of the sunscreen formulations, 20 mg of sunscreen was pipetted in a spiral fashion outwards, starting from the center, on a HelioScreen HelioPlate HD6 (Labsphere). The sunscreen was spread manually, with an index finger wearing a finger cot pre-wet with a small amount of sunscreen. The sunscreen was spread in a zigzag fashion from top to bottom, then the square plate was turned 90° clockwise and the motion was repeated. The spreading was performed for a total of 4 times, each starting from a different side of the square plate. The plate was left to dry for at least 15-30 mins before SPF measurements were taken. For each sunscreen, three different plates were used. The UV-2000S (Labsphere) was used to measure the SPF of each plate, using the COLIPA method and measuring five different spots on each plate.

*Results and discussion*

In the initial studies, the sunscreen containing the FAS-Janus particle (at 5 wt.% particle loading) was compared against two controls: one made with untreated TiO$_2$, and the other one made with TiO$_2$ fully coated with FAS. They were evaluated based on their SPF rating and the results are as shown in (Fig. 28). Among the three sunscreens, the one made with Janus FAS particles showed the highest SPF rating, and is therefore considered to have the best sunscreen performance.

In the subsequent studies, the SPF testing was extended to other sunscreens made with different particles (FAS, FAS-Janus, OTES and OTES-Janus, with 5 wt.% particle loading). Fig. 29 shows the relative SPF values attained. The Blank formulation (base formulation with no particles, but with 5 wt.% water in replacement of the particles) had a base SPF of around 0.95. Untreated particles gave an SPF of 2.87, which was higher than the fully coated FAS particles (SPF 2.66) and OTES particles (SPF 2.23). The Janus particles showed the best SPF performances, with both FAS-Janus (SPF 3.46) and OTES-Janus (SPF 2.97) outperforming FAS, OTES and untreated particles. Across all formulations, FAS-Janus showed the highest SPF.

To evaluate further, different percent loading of TiO$_2$ particles in the sunscreen and its impact on SPF was examined. In addition to 5.0 wt.% particle loading, 2.5% and 10.0% particle loading of TiO$_2$ was examined, and water was added and reduced respectively, *quantum satis.* FAS, FAS-Janus, OTES, and OTES-Janus were used for these formulations. The resulting SPF values are shown in Fig. 30. Consistent with the observations as shown in Fig. 29, the SPF ratings for FAS-Janus (triangle) and OTES-Janus (star) outperformed their fully coated counterparts at 10.0% particle loading, while at 2.5% particle loading only FAS-Janus showed a significant better SPF than the other three formulations. Overall, Janus particles showed better SPF performance. A linear fit of the three data points for each sunscreen (Fig. 30) produced slopes of 0.36 for FAS, 0.31 for OTES, 0.44 for FAS-Janus, and 0.49 for OTES-Janus, showing greater improvements in SPF with increased particle loading when Janus particles were used. In general, increased particle loading leads to increased SPF due to the higher amount of $TiO_2$ particles available to block UV. However, this also gives rise to a whitish appearance when the sunscreen is applied on the skin, which is not aesthetically favorable. Using Janus particles could circumvent this issue through the use of less particles to achieve equivalent SPF values (as fully coated FAS or OTES-particles).

**Example 8. Self-assembly behaviour of the as-prepared Janus particles on synthetic skin substrates**

To evaluate the assembly behaviour of the particles, the sunscreen formulations were applied on a synthetic skin substrate (VITROSKIN, IMS Inc.). To prepare the VITROSKIN for application, the VITROSKIN was cut into pieces measuring 6.2 cm x 9.0 cm, and incubated for 24 hrs in a sealed chamber containing 15% weight glycerin in water at ambient temperature. 100 $\mu$L of each sunscreen was pipetted across a 6.2 cm x 8.0 cm area on the VITROSKIN, and a finger cot was used to apply the sunscreen, first in a circular motion starting from one corner, then in a zigzag motion. The VITROSKIN was then left to dry in ambient conditions for 30 mins, before cutting into smaller samples to be viewed under FESEM (JEOL JSM-7600F) at 10 keV accelerating voltage, in secondary electron imaging mode. An approximately 5 nm of gold was sputtered over the samples prior to visualisation. Five different sunscreen formulations were examined: untreated, FAS, FAS-Janus, OTES, and OTES-Janus (at 5.0 wt.% particle loading).

Fig. 31 shows the spread of sunscreen over the VITROSKIN as visualised under FESEM. In general, the sunscreen formulation consisted of the main sunscreen particles ($TiO_2$) and polymers to build up the bulk. The interactions between the bulk and the particles are essential in how the particles are distributed across skin, as the bulk serves as a carrier with which the sunscreen is applied. The FESEM images were evaluated on the basis as to how evenly the sunscreen (particles and bulk formulation) had spread across the VITROSKIN.

Across formulations, sunscreen containing the untreated $TiO_2$ particles (Fig. 31a) had spread as thick multilayers over VITROSKIN, while FAS (Fig. 31b) and OTES (Fig. 31d) showed many bare areas (representing skin unprotected by sunscreen formulation). For the sunscreens containing the Janus particles, few bare areas were seen. The suncreen containing the OTES-Janus particles showed some multilayers (Fig. 31e), while FAS-Janus (Fig. 31c) showed the most optimal spread behaviour among all sunscreens: the formulation appeared even, existing as thin layers, and achieved adequate coverage over the VITROSKIN with few bare areas. Overall, based on the appearance of how the sunscreen had spread, the Janus sunscreens showed most optimal spreading behaviour, starting with FAS-Janus, then OTES-Janus, followed by Untreated, and finally followed by FAS and OTES. This observation is consistent with the SPF results as shown in Fig. 29, that a more even application of sunscreen leads to higher SPF.

As such, the use of the Janus particles of the current invention in sunscreen has been shown to provide an even application of sunscreen, which not only more effectively protects the skin from UV damage, but also potentially acts as a physical shield against small particulate matter in the environment. The penetration of dust and other pollutants common to urban areas into the skin may be prevented by the physical barrier imposed by the even spread of sunscreen.

**Claims**

1.  A Janus particle comprising:

   a core inorganic photocatalytic particle having a surface with a first region and a second region ; and
   a $C_8$ to $C_{26}$ alkylsilane or a fluoroalkylsilane coating that is susceptible to photo-degradation, which coating is covalently bound to the surface of the core inorganic photocatalytic particle where present, wherein:

      the coating fully coats the entire surface of the first region and the second region is coated by a residue formed by photo-degradation of the coating, such that;
      the first region displays hydrophobic or hydrophobic and oleophobic properties; and
      the second region displays amphiphilic properties.

2.  The particle according to Claim 1, wherein:

   the core inorganic photocatalytic particle is selected from one or more of $CeO_2$, $SnO_2$, $Nb_2O_5$, $WO_3$, $Fe_2O_3$, $Ta_2O_3$, $CuO$, $NiO$, $Cr_2O_3$, $RuO_2$, $TiO_2$, $ZnO$, and $Cu_2O$; and/or
   the core inorganic photocatalytic particle is itself a hybrid core-shell particle having a core selected from one or more of silica, a polymer, a ceramic, a metal and an alloy and a shell comprising one or more of $CeO_2$, $SnO_2$, $Nb_2O_5$, $WO_3$, $Fe_2O_3$, $Ta_2O_3$, $CuO$, $NiO$, $Cr_2O_3$, $RuO_2$, $TiO_2$, $ZnO$, and $Cu_2O$.

3. The particle according to Claim 1 or Claim 2, wherein the core inorganic photocatalytic particle is selected from one or more of $TiO_2$, $ZnO$, and $Cu_2O$, optionally wherein the core inorganic photocatalytic particle is $TiO_2$.

4. The particle according to any one of the preceding claims, wherein the first region forms from 30 to 70% of the surface area of the Janus particle and the second region forms from 70 to 30% of the surface area of the Janus particle.

5. The particle according to Claim 4, wherein the first region forms from 40 to 60% of the surface area of the Janus particle and the second region forms from 60 to 40% of the surface area of the Janus particle, optionally wherein the first and second region form 50% of the surface area of the Janus particle.

6. The particle according to any one of the preceding claims, wherein the Janus particles, in the form of a compact film, have a water contact angle of from 30 to 140°.

7. A method of manufacturing a Janus particle, the method comprising the steps of:

(a) providing an aqueous dispersion of precursor Janus particles comprising a core inorganic photocatalytic particle, wherein the core inorganic photocatalytic particle has a surface that is fully coated by a $C_8$ to $C_{26}$ alkylsilane or a fluoroalkylsilane coating that is susceptible to photo-degradation, which coating is covalently bound to the surface of the core inorganic photocatalytic particle; and
(b) subjecting the aqueous dispersion to irradiation by ultra-violet light for a period of time to provide Janus particles comprising:

the core inorganic photocatalytic particle having a surface with a first region and a second region; and
a $C_8$ to $C_{26}$ alkylsilane or a fluoroalkylsilane coating that is susceptible to photo-degradation, which coating is covalently bound to the surface of the core inorganic photocatalytic particle where present, wherein:

the coating fully coats the entire surface of the first region and the second region is coated by a residue formed by photo-degradation of the coating, such that;
the first region displays hydrophobic or hydrophobic and oleophobic properties; and the second region displays amphiphilic properties, wherein
the period of time is from 30 minutes to 2 hours;
the ultra-violet light is provided at a wavelength of from 100 to 400 nm; and
the ultra-violet light is provided at an irradiance value of from 50 to 150 mW/cm$^2$.

8. The method according to Claim 7, wherein:

(AA) the period of time is 1 hour; and/or
(BB) the ultra-violet light is provided at a wavelength of 365 nm.

9. The method according to Claim 7 or Claim 8, wherein the ultra-violet light is provided at an irradiance value of from 70 to 140 mW/cm$^2$, optionally 72 mW/cm$^2$ or 136 mW/cm$^2$.

10. The method according to any one of Claims 7 to 9, wherein:

(AAA) the precursor Janus particles are prepared by reacting core inorganic photocatalytic particles with one or more of a $C_8$ to $C_{26}$ alkyltrialkoxysilane or a fluoroalkyltrialkoxysilane to provide the precursor Janus particles, optionally wherein the core inorganic photocatalytic particles are reacted with one or more of octyltriethoxysilane, hexadecyltrimethoxysilane, and 1H,1H,2H,2H-perfluoro-decyltriethoxysilane; and/or
(BBB) the precursor Janus particles are partially coated in a wax that covers from 30 to 70%, such as from 60 to 40%, such as 50%, of the surface area of the precursor Janus particle, optionally wherein after step (b) the wax is removed from the particles using a suitable organic solvent or the precursor Janus particles are provided in the form of water-in-oil Pickering emulsion droplets that are dispersed into water to form the aqueous dispersion of precursor Janus particles.

11. The method according to any one of Claims 7 to 10, wherein:

the core inorganic photocatalytic particle is selected from one or more of CeO$_2$, SnO$_2$, Nb$_2$O$_5$, WO$_3$, Fe$_2$O$_3$, Ta$_2$O$_3$, CuO, NiO, Cr$_2$O$_3$, RuO$_2$, TiO$_2$, ZnO, and Cu$_2$O; and/or

the core inorganic photocatalytic particle is a hybrid core-shell particle having a silica core and a shell comprising one or more of CeO$_2$, SnO$_2$, Nb$_2$O$_5$, WO$_3$, Fe$_2$O$_3$, Ta$_2$O$_3$, CuO, NiO, Cr$_2$O$_3$, RuO$_2$, TiO$_2$, ZnO, and Cu$_2$O, optionally wherein the core inorganic photocatalytic particle is selected from one or more of TiO$_2$, ZnO, and Cu$_2$O, optionally wherein the core inorganic photocatalytic particle is TiO$_2$.

12. The method according to any one of Claims 7 to 11, wherein the coating is 1H,1H,2H,2H-perfluorodecylsilane.

13. A sunscreen formulation comprising Janus particles as described in any one of Claims 1 to 6 and a diluent, carrier or excipient.

**Patentansprüche**

1. Janus-Teilchen, umfassend:

ein anorganisches photokatalytisches Kernteilchen, das eine Oberfläche mit einer ersten Region und einer zweiten Region aufweist; und

eine C$_8$- bis C$_{26}$-Alkylsilan- oder eine Fluoralkylsilan-Beschichtung, die für Photodegradation empfindlich ist, wobei die Beschichtung kovalent an die Oberfläche des anorganischen photokatalytischen Kernteilchens, sofern vorhanden, gebunden ist, wobei:

die Beschichtung die gesamte Oberfläche der ersten Region vollständig beschichtet und die zweite Region mit einem durch Photodegradation der Beschichtung gebildeten Rückstand beschichtet ist, so dass; die erste Region hydrophobe oder hydrophobe und oleophobe Eigenschaften aufzeigt; und die zweite Region amphiphile Eigenschaften aufzeigt.

2. Teilchen nach Anspruch 1, wobei:

das anorganische photokatalytische Kernteilchen aus einem oder mehreren von CeO$_2$, SnO$_2$, Nb$_2$O$_5$, WO$_3$, Fe$_2$O$_3$, Ta$_2$O$_3$, CuO, NiO, Cr$_2$O$_3$, RuO$_2$, TiO$_2$, ZnO und Cu$_2$O ausgewählt ist; und/oder das anorganische photokatalytische Kernteilchen selbst ein hybrides Kern-Schale-Teilchen ist, das einen Kern, der aus einem oder mehreren von Siliciumdioxid, einem Polymer, einer Keramik, einem Metall und einer Legierung ausgewählt ist, und eine Schale, die eines oder mehrere von CeO$_2$, SnO$_2$, Nb$_2$O$_5$, WO$_3$, Fe$_2$O$_3$, Ta$_2$O$_3$, CuO, NiO, Cr$_2$O$_3$, RuO$_2$, TiO$_2$, ZnO und Cu$_2$O umfasst, aufweist.

3. Teilchen nach Anspruch 1 oder Anspruch 2, wobei das anorganische photokatalytische Kernteilchen aus einem oder mehreren von TiO$_2$, ZnO und Cu$_2$O ausgewählt ist, wobei wahlweise das anorganische photokatalytische Kernteilchen TiO$_2$ ist.

4. Teilchen nach einem der vorhergehenden Ansprüche, wobei die erste Region 30 bis 70 % des Oberflächenbereichs des Janus-Teilchens bildet und die zweite Region 70 bis 30 % des Oberflächenbereichs des Janus-Teilchens bildet.

5. Teilchen nach Anspruch 4, wobei die erste Region 40 bis 60 % des Oberflächenbereichs des Janus-Teilchens bildet und die zweite Region 60 bis 40 % des Oberflächenbereichs des Janus-Teilchens bildet, wobei wahlweise die erste und zweite Region 50 % des Oberflächenbereichs des Janus-Teilchens bilden.

6. Teilchen nach einem der vorhergehenden Ansprüche, wobei die Janus-Teilchen, in der Form eines kompakten Films, einen Wasserkontaktwinkel von 30 bis 140° aufweisen.

7. Verfahren zum Herstellen eines Janus-Teilchens, das Verfahren die folgenden Schritte umfassend:

(a) Bereitstellen einer wässerigen Dispersion von Janus-Vorläuferteilchen, die ein anorganisches photokatalytisches Kernteilchen umfasst, wobei das anorganische photokatalytische Kernteilchen eine Oberfläche hat, die mit einer C$_8$- bis C$_{26}$-Alkylsilan- oder einer Fluoralkylsilan-Beschichtung, die für Photodegradation empfindlich ist, vollständig beschichtet ist, wobei die Beschichtung kovalent an die Oberfläche des anorganischen photokatalytischen Kernteilchens gebunden ist; und

(b) Aussetzen der wässerigen Dispersion einer Bestrahlung mit ultraviolettem Licht für eine Zeitdauer, um Janus-Teilchen bereitzustellen, umfassend:

das anorganische photokatalytische Kernteilchen, das eine Oberfläche mit einer ersten Region und einer zweiten Region aufweist; und
eine $C_8$- bis $C_{26}$-Alkylsilan- oder eine Fluoralkylsilan-Beschichtung, die für Photodegradation empfindlich ist, wobei die Beschichtung kovalent an die Oberfläche des anorganischen photokatalytischen Kernteilchens, sofern vorhanden, gebunden ist, wobei:

die Beschichtung die gesamte Oberfläche der ersten Region vollständig beschichtet und die zweite Region mit einem durch Photodegradation der Beschichtung gebildeten Rückstand beschichtet ist, so dass;
die erste Region hydrophobe oder hydrophobe und oleophobe Eigenschaften aufzeigt; und die zweite Region amphiphile Eigenschaften aufzeigt, wobei
die Zeitdauer 30 Minuten bis 2 Stunden beträgt;
das ultraviolette Licht bei einer Wellenlänge von 100 bis 400 nm bereitgestellt wird; und
das ultraviolette Licht bei einem Bestrahlungswert von 50 bis 150 mW/cm$^2$ bereitgestellt wird.

8. Verfahren nach Anspruch 7, wobei:

(AA) die Zeitdauer 1 Stunde beträgt; und/oder
(BB) das ultraviolette Licht bei einer Wellenlänge von 365 nm bereitgestellt wird.

9. Verfahren nach Anspruch 7 oder Anspruch 8, wobei das ultraviolette Licht bei einem Bestrahlungswert von 70 bis 140 mW/cm$^2$ bereitgestellt wird, wahlweise 72 mW/cm$^2$ oder 136 mW/cm$^2$.

10. Verfahren nach einem der Ansprüche 7 bis 9, wobei:

(AAA) die Janus-Vorläuferteilchen durch Umsetzen von anorganischen photokatalytischen Kernteilchen mit einem oder mehreren eines $C_8$- bis $C_{26}$-Alkyltrialkoxysilans oder eines Fluoralkyltrialkoxysilans angefertigt werden, um die Janus-Vorläuferteilchen bereitzustellen, wobei wahlweise die anorganischen photokatalytischen Kernteilchen mit einem oder mehreren von Octyltriethoxysilan, Hexadecyltrimethoxysilan und 1H,1H,2H,2H-Perfluordecyltriethoxysilan umgesetzt werden; und/oder
(BBB) die Janus-Vorläuferteilchen teilweise mit einem Wachs beschichtet sind, das 30 bis 70 %, wie 60 bis 40 %, wie 50 %, des Oberflächenbereichs des Janus-Vorläuferteilchens bedeckt, wobei wahlweise nach Schritt (b) das Wachs von den Teilchen unter Verwendung eines geeigneten organischen Lösungsmittels entfernt wird oder die Janus-Vorläuferteilchen in der Form von Wasser-in-Öl-Pickering-Emulsionströpfchen bereitgestellt werden, die in Wasser dispergiert werden, um die wässerige Dispersion von Janus-Vorläuferteilchen zu bilden.

11. Verfahren nach einem der Ansprüche 7 bis 10, wobei:

das anorganische photokatalytische Kernteilchen aus einem oder mehreren von $CeO_2$, $SnO_2$, $Nb_2O_5$, $WO_3$, $Fe_2O_3$, $Ta_2O_3$, CuO, NiO, $Cr_2O_3$, $RuO_2$, $TiO_2$, ZnO und $Cu_2O$ ausgewählt ist; und/oder
das anorganische photokatalytische Kernteilchen ein hybrides Kern-Schale-Teilchen ist, das einen Siliciumdioxid-Kern und eine Schale, die eine oder mehrere von $CeO_2$, $SnO_2$, $Nb_2O_5$, $WO_3$, $Fe_2O_3$, $Ta_2O_3$, CuO, NiO, $Cr_2O_3$, $RuO_2$, $TiO_2$, ZnO und $Cu_2O$ umfasst, aufweist,
wobei wahlweise das anorganische photokatalytische Kernteilchen aus einem oder mehreren von $TiO_2$, ZnO und $Cu_2O$ ausgewählt ist, wobei wahlweise das anorganische photokatalytische Kernteilchen $TiO_2$ ist.

12. Verfahren nach einem der Ansprüche 7 bis 11, wobei die Beschichtung 1H,1H,2H,2H-Perfluordecylsilan ist.

13. Sonnenschutzformulierung, umfassend Janus-Teilchen, wie in einem der Ansprüche 1 bis 6 beschrieben, und ein Verdünnungsmittel, einen Träger oder einen Füllstoff.

**Revendications**

1. Particule de Janus comprenant :

   une particule photocatalytique inorganique centrale présentant une surface avec une première région et une seconde région ; et
   un revêtement d'alkylsilane en $C_8$ à $C_{26}$ ou de fluoroalkylsilane qui est sensible à la photodégradation, ledit revêtement étant lié de manière covalente à la surface de la particule photocatalytique inorganique centrale lorsqu'elle est présente, dans laquelle :
   le revêtement recouvre totalement toute la surface de la première région et la seconde région est revêtue par un résidu formé par la photodégradation du revêtement, de sorte que :

   la première région affiche des propriétés hydrophobes ou hydrophobes et oléophobes ; et
   la seconde région affiche des propriétés amphiphiles.

2. Particule selon la revendication 1, dans laquelle :

   la particule photocatalytique inorganique centrale est sélectionnée parmi un ou plusieurs éléments parmi le $CeO_2$, le $SnO_2$, le $Nb_2O_5$, le $WO_3$, le $Fe_2O_3$, le $Ta_2O_3$, le CuO, le NiO, le $Cr_2O_3$, le $RuO_2$, le $TiO_2$, le ZnO et le $Cu_2O$ ; et/ou
   la particule photocatalytique inorganique centrale est elle-même une particule de type noyau-enveloppe hybride présentant un noyau sélectionné parmi un ou plusieurs éléments parmi la silice, un polymère, une céramique, un métal et un alliage et une enveloppe comprenant un ou plusieurs des éléments parmi le $CeO_2$, le $SnO_2$, le $Nb_2O_5$, le $WO_3$, le $Fe_2O_3$, le $Ta_2O_3$, le CuO, le NiO, le $Cr_2O_3$, le $RuO_2$, le $TiO_2$, le ZnO et le $Cu_2O$.

3. Particule selon la revendication 1 ou la revendication 2, dans laquelle la particule photocatalytique inorganique centrale est sélectionnée parmi un ou plusieurs éléments parmi le $TiO_2$, le ZnO et le $Cu_2O$, facultativement dans laquelle la particule photocatalytique inorganique centrale est le $TiO_2$.

4. Particule selon l'une quelconque des revendications précédentes, dans laquelle la première région forme de 30 à 70 % de l'aire de surface de la particule de Janus et la seconde région forme de 70 à 30 % de l'aire de surface de la particule de Janus.

5. Particule selon la revendication 4, dans laquelle la première région forme de 40 à 60 % de l'aire de surface de la particule de Janus et la seconde région forme de 60 à 40 % de l'aire de surface de la particule de Janus, facultativement dans laquelle la première et la seconde région forment 50 % de l'aire de surface de la particule de Janus.

6. Particule selon l'une quelconque des revendications précédentes, dans laquelle les particules de Janus, sous la forme d'un film compact, présentent un angle de contact avec l'eau de 30 à 140°.

7. Procédé de fabrication d'une particule de Janus, le procédé comprenant les étapes consistant à :

   (a) fournir une dispersion aqueuse de particules de Janus précurseurs comprenant une particule photocatalytique inorganique centrale,
   dans lequel la particule photocatalytique inorganique centrale présente une surface qui est totalement revêtue d'un revêtement d'alkylsilane en $C_8$ à $C_{26}$ ou de fluoroalkylsilane qui est sensible à la photodégradation, ledit revêtement étant lié de manière covalente à la surface de la particule photocatalytique inorganique centrale ; et
   (b) soumettre la dispersion aqueuse à un rayonnement par une lumière ultraviolette pendant un laps de temps pour fournir des particules de Janus comprenant :

   la particule photocatalytique inorganique centrale présentant une surface avec une première région et une seconde région ; et
   un revêtement d'alkylsilane en $C_8$ à $C_{26}$ ou de fluoroalkylsilane qui est sensible à la photodégradation, ledit revêtement étant lié de manière covalente à la surface de la particule photocatalytique inorganique centrale, lorsqu'elle est présente, dans lequel :
   le revêtement recouvre totalement toute la surface de la première région et la seconde région est revêtue par un résidu formé par la photodégradation du revêtement, de sorte que :

la première région affiche des propriétés hydrophobes ou hydrophobes et oléophobes ; et la seconde région affiche des propriétés amphiphiles, dans lequel :

le laps de temps est de 30 minutes à 2 heures ;
la lumière ultraviolette est fournie à une longueur d'onde de 100 à 400 nm ; et
la lumière ultraviolette est fournie à une valeur d'irradiance de 50 à 150 mW/cm$^2$.

8. Procédé selon la revendication 7, dans lequel

(AA) le laps de temps est d'1 heure ; et/ou
(BB) la lumière ultraviolette est fournie à une longueur d'onde de 365 nm.

9. Procédé selon la revendication 7 ou la revendication 8, dans lequel la lumière ultraviolette est fournie à une valeur d'irradiance de 70 à 140 mW/cm$^2$, facultativement 72 mW/cm$^2$ ou 136 mW/cm$^2$.

10. Procédé selon l'une quelconque des revendications 7 à 9, dans lequel :

(AAA) les particules de Janus précurseurs sont préparées en faisant réagir des particules photocatalytiques inorganiques centrales avec un ou plusieurs d'un alkyltrialcoxysilane en $C_8$ à $C_{26}$ ou un fluoroalkyltrialcoxysilane pour fournir les particules de Janis précurseurs, facultativement dans lequel les particules photocatalytiques inorganique centrales sont mises en réaction avec un ou plusieurs éléments parmi l'octyltriéthoxysilane, l'hexadécyltriméthoxysilane et le 1H, 1H, 2H, 2H-perfluoro-décyltriéthoxysilane ; et/ou
(BBB) les particules de Janus précurseurs sont partiellement revêtues d'une cire qui recouvre de 30 à 70 %, comme de 60 à 40 %, comme 50 %, de l'aire de surface de la particule de Janus précurseur, facultativement dans lequel après l'étape (b), la cire est enlevée des particules en utilisant un solvant organique adapté ou les particules de Janus précurseurs sont fournies sous la forme de gouttelettes d'émulsion Pickering eau-en-huile qui sont dispersées dans l'eau pour former la dispersion aqueuse de particules de Janus précurseurs.

11. Procédé selon l'une quelconque des revendications 7 à 10, dans lequel :

la particule photocatalytique inorganique centrale est sélectionnée parmi un ou plusieurs éléments parmi le $CeO_2$, le $SnO_2$, le $Nb_2O_5$, le $WO_3$, le $Fe_2O_3$, le $Ta_2O_3$, le CuO, le NiO, le $Cr_2O_3$, le $RuO_2$, le $TiO_2$, le ZnO et le $Cu_2O$ ; et/ou
la particule photocatalytique inorganique centrale est une particule de type noyau-enveloppe hybride présentant un noyau de silice et une enveloppe comprenant un ou plusieurs éléments parmi le $CeO_2$, le $SnO_2$, le $Nb_2O_5$, le $WO_3$, le $Fe_2O_3$, le $Ta_2O_3$, le CuO, le NiO, le $Cr_2O_3$, le $RuO_2$, le $TiO_2$, le ZnO et le $Cu_2O$, facultativement dans lequel la particule photocatalytique inorganique centrale est sélectionnée parmi le $TiO_2$, le ZnO et le $Cu_2O$, facultativement dans laquelle la particule photocatalytique inorganique centrale est le $TiO_2$.

12. Procédé selon l'une quelconque des revendications 7 à 11, dans lequel le revêtement est le 1H,1H,2H,2H-perfluoro-décylsilane.

13. Formulation d'écran solaire comprenant des particules de Janus comme décrit dans l'une quelconque des revendications 1 à 6 et un diluant, support ou excipient.

**FIG. 1**

FIG. 2

**FIG. 3**

**FIG. 4**

(a)　　　　　　　(b)　　　　　　　(c)

**FIG. 5**

(a)　　　　　　　(b)

**FIG. 6**

**(a)**　　　　　　**(b)**

**(c)**　　　　　　**(d)**

**(e)**　　　　　　**(f)**

**FIG. 7**

**FIG. 8**

**FIG. 9**

**FIG. 10**

**FIG. 11**

**FIG. 12**

**FIG. 13**

FIG. 14

FIG. 15

FIG. 16

FIG. 17

FIG. 18

FIG. 19

FIG. 20

FIG. 21

FIG. 22

FIG. 23

FIG. 24

FIG. 25

**FIG. 26**

**FIG. 27**

**FIG. 28**

**FIG. 29**

**FIG. 30**

FIG. 31

# EP 3 829 756 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20080234394 A1

### Non-patent literature cited in the description

- **HU, J. et al.** *Chem. Soc. Rev.*, 2012, vol. 41, 4356-4378
- **PERRO, A. et al.** *, J. Mater. Chem.*, 2005, vol. 15, 3745-3760
- **JIANG, S.;** ; **GRANICK, S.** *Langmuir*, 2008, vol. 24, 2438-2445
- **TAKAHARA, Y. K. et al.** *J. Am. Chem. Soc.*, 2005, vol. 127, 6271-6275
- **WIECHERS, S et al.** *Cosmetics & Toiletries*, 2013, vol. 128 (5), 2-6
- **G. L. WITUCKI.** *J. Coat. Technol.*, 1993, vol. 65, 57
- **D.-F. ZHANG et al.** *J. Mater. Chem.*, 2009, vol. 19, 5220-5225
- **E. HOQUE et al.** *J. Phys. Chem. C*, 2007, vol. 111, 3956-3962
- **P. B. KOWALCZUK et al.** *Colloids Surf. Physicochem. Eng. Aspects*, 2012, vol. 393, 81-85
- **BINKS, B. et al.** *Langmuir*, 2000, vol. 16, 2539-2547
- **YE, T. et al.** *J. Phys. Chem. B*, 2005, vol. 109, 9927-9938
- **E. UKAJI et al.** *Appl. Surf. Sci*, 2007, vol. 254, 563-569
- **S. PAZOKIFARD et al.** *, Adv. Powder Technol.*, 2012, vol. 23, 428-436
- **J.-D. BRASSARD et al.** *Appl. Sci.*, 2012, vol. 2, 453-464
- **X. WANG et al.** *Holzforschung*, 2013, vol. 67, 667-672
- **T. MONDE et al.** *J. Ceram. Soc. Jpn.*, 1996, vol. 104, 682-684
- **G. J. KLUTH et al.** *Langmuir*, 1997, vol. 13, 3775-3780
- **CAMPOS, R. et al.** *Langmuir*, 2011, vol. 27, 10206-10215
- **K. C. GRABAR et al.** *Anal. Chem.*, 1995, vol. 67, 735-743
- **CHU, C.-Y.;** ; **HUANG, M. H.** *J. Mat. Chem. A*, 2017, vol. 5, 15116-15123
- **B. BINKS** ; **P. FLETCHER**. *Langmuir*, 2001, vol. 17, 4708-4710
- **T. ONDARÇUHU et al.** *J. Phys.*, 1990, vol. 51, 1527-1536
- **Y. LAI et al.** *Part. Part. Syst. Char.*, 2015, vol. 32, 355-363
- **S. JIANG** ; **S. GRANICK**. Janus balance of amphiphilic colloidal particles. AIP, 2007
- **WIECHERS, S. et al.** *Cosmetics & Toiletries*, 2013, vol. 128, 2-6